# EUROPÄISCHE PATENTSCHRIFT

(11) **EP 1 139 897 B1**
(45) Veröffentlichungstag und Bekanntmachung des Hinweises auf die Patenterteilung: **04.05.2005**
(21) Anmeldenummer: 99966974.0
(22) Anmeldetag: 17.12.1999
(51) Int. Cl.: A61B 18/14, A61B 18/22

(54) **ELEKTRODENANORDNUNG FÜR EIN CHIRURGISCHES INSTRUMENT ZUR ELEKTROTHERMISCHEN KOAGULATION IM GEWEBE**
ELECTRODE ASSEMBLY FOR A SURGICAL INSTRUMENT PROVIDED FOR CARRYING OUT AN ELECTROTHERMAL COAGULATION OF TISSUE
ENSEMBLE ELECTRODE POUR UN INSTRUMENT CHIRURGICAL SERVANT A LA COAGULATION ELECTROTHERMIQUE DE TISSU

(30) Priorität: 18.12.1998 DE 19858599
(43) Veröffentlichungstag der Anmeldung: 10.10.2001
(73) Patentinhaber: Celon AG Medical Instruments, 14513 Teltow (DE)
(72) Erfinder: DESINGER, Kai, 12157 Berlin (DE)
(74) Vertreter: von Oppen, Joachim, Dipl.-Ing.
(86) Internationale Anmeldenummer: PCT/EP1999/010079
(87) Internationale Veröffentlichungsnummer: WO 2000/036985

(56) Entgegenhaltungen:
- WO-A-97/00647
- DE-A- 19 739 699

## Beschreibung

Die Erfindung betrifft eine Elektrodenanordnung fürein chirurgisches Instrument zur elektrothermischen Koagulation von Gewebe, welches einen Frontzylinder am distalen Ende des Instrumentes mit einer distalen Spitze enthält, mit einem proximal an Frontzylinder anschließenden Träger, und mit zwei Elektroden, die an eine Wechselspannungsquelle anschließbar sind.

Die Anwendung hochfrequenter Wechselströme (beispielsweise im Frequenzbereich von 300 KHz bis 2 MHz) zur Erzeugung hoher Temperaturen zu Gewebekoagulation und zur Gewebetrennung zu verwenden ist in der Chirurgie seit langem bekannt. In der Praxis werden zur Einbringung des HF-Stromes in das Gewebe sogenannte mono-polare Elektrodenanordnungen oder bipolare Elektrodenanordnungen eingesetzt.

Bei den monopolaren Anordnungen wird eine Elektrode - auch als Neutralelektrode bezeichnet - als großflächige Elektrode in der Nähe des Behandlungsortes auf die Haut des Patienten angesetzt und dort fixiert und geerdet, bzw. mit Masse verbunden. Eine zweite vom Operateur gehandhabte Elektrode - auch als Aktivelektrode bezeichnet - ist mit der Wechselspannungsquelle verbunden. Die Elektrode ist in ihrer Form an die jeweilige Anwendung, insbesondere an die Größe des zu behandelnden Gewebebereiches so angepasst, dass sowohl die Operationsdauer als auch die thermische Belastung des betroffenen Organes bzw. Körperbereiches vertretbar sind und nur den gewünschten Gewebebereich koagulieren.

Bei Anordnungen zur bipolaren HF-Thermotherapie sind beide Elektroden mit einem HF-Generator verbunden und in miteinander festgelegte Abmessungen, beispielsweise auf einem isolierenden Träger angeordnet und werden vom Operateur in unmittelbarer Nähe der Behandlungsstelle platziert und in der Regel auch aktiv geführt.

Aus der WO 97/17009 ist eine bipolare Elektrodenanordnung mit einem Flüssigkeitskanal bekannt, über den Spülflüssigkeit in den Eingriffsbereich eingebracht werden kann. Zwei oder drei Elektroden sind als Konusabschnitt auf einer konusförmigen distalen Spitze des Instrumentes angeordnet, die in das Gewebe eingeführt werden kann, wobei das elektromagnetische HF-Feld sich zwischen den Elektroden ausbildet und das umgebene Gewebe koagulieren soll.

Aus der WO 96/34569 sowie den im zugehörigen internationalen Recherchenbericht genannten Dokumenten sind Systeme und Verfahren zur Koagulation von Körpergewebe unter Einhaltung einer vorberechneten maximalen Gewebstemperatur bekannt, bei denen während der eigentlichen Gewebskoagulation eine Fluidkühlung oder thermoelektrische Kühlung vorgesehen ist. Diese bekannten Anordnungen sind zur Einführung in Körperhöhlen über natürliche Zugänge gedacht.

Aus der US 4,832,048 sowie aus der WO 95/10320 der WO 99/11186 oder der EP 96 945 879.3 und der WO98/1961 der WO96/18349 und der WO81/03272 und der DE 197 39 699 sind weiter chirurgische Instrumente bekannt, die mittels einer bipolaren Elektrodenanordnung Gewebe mittels HF-Thermotherapie behandeln.

Die bekannten chirurgischen Instrumente zur bipolaren HF-Thermotherapie sind oftmals aufwendig in der Herstellung, und sie besitzen für die verschiedenen Anwendungsgebiete oftmals Nachteile, die oftmals zu einer lokal ungenauen Gewebebehandlung führen, die insbesondere das zu behandelnde Gewebe teilweise nicht erreicht, bzw. gutartiges Gewebe thermisch überlastet.

Aufgabe der Erfindung ist es daher, eine Elektrodenanordnung für ein chirurgisches Instrument der eingangs genannten Art derart weiterzubilden, dass es einfach herstellbar und einsetzbar ist und eine präzise lokalisierbare Behandlung des Gewebes - bei gleichzeitiger Schonung des umgebenden gesunden Gewebes - ermöglicht.

Diese Aufgabe wird erfindungsgemäß gelöst durch eine Elektrodenanordnung für ein chirurgisches Instrument zur elektrothermischen Koagulation von Gewebe wie sie im Anspruchssatz definiert ist. Die Anordnung enthält einen elektrisch leitenden Frontzylinder am distalen Ende des Instruments, mit einer distalen Spitze, und mit einer zylindrischen ersten Elektrode, einen proximal an den Frontzylinder anschließenden rohrförmigen Außenleiter mit einer zylindrischen zweiten Elektrode, ein Isolatorelement zwischen dem Frontzylinder und dem Außenleiter, wobei die Elektroden an eine Wechselspannungsquelle anschließbar sind, gekennzeichnet durch einen stabförmigen Innenleiter in dem Außenleiter und einen Isolationsschlauch zwischen dem Innenleiter und dem Außenleiter. Das Isolatorelement ist als Ringkörper mit einer vorgegebenen axialem Länge ausgebildet. Der Innenleiter läuft durch den Ringkörper. Frontzylinder und Außenleiter liegen an den Stirnseiten des Ringkörpers an.

Die Vorteile der Erfindung liegen insbesondere darin, dass die Elektrodenanordnung besonders einfach aufgebaut ist, wobei nämlich der Frontzylinder eine Elektrode bildet, und dass der über ein Isolatorelement isolierte, anschließende Außenleiter die zweite Elektrode bildet, so dass die Wechselspannungsquelle über den stabförmigen Innenleiter, der durch einen Isolationsschlauchaußenleiter getrennt ist, an den Frontzylinder und von außen direkt an den Außenleiter anschließbar ist.

Das Isolationselement besitzt zwischen dem Frontzylinder und dem Außenleiter eine radiale Trennwand, die außen in eine zylindrische Hüllwand übergeht, welche den Frontzylinder und/oder den Außenleiter eng anliegend über einen vorgegebenen axialen Längsabschnitt umgibt. Der Innenleiter ist durch diese Trennwand des Isolationselernentes hindurch mit dem Frontzylinder verbindbar. Bevorzugt wird zwischen Frontzylinder und dem Innenleiter eine lösbare Schraubverbindung verwirklicht, bei der der Innenleiter an seinem distalen Ende ein Außengewinde trägt, welches mit einem entsprechenden axialen Innengewinde im Frontzylinder verschraubbar ist. Die Vorteile dieser Ausführungsform liegen insbesondere darin, dass das becherartige Isolatorelement als eine Isolationsschicht direkt auf den metallischen Frontzylinder und/oder den metallischen Außenleiter so aufgebracht werden kann, dass die Trennfläche zwischen Frontzylinder und Außenleiter und ein sich hieran axial anschließender Längsabschnitt der Außenfläche eine Beschichtung mit einem Isolatormaterial aufweist. Eine derartige Beschichtung lässt sich beispielsweise durch Eloxieren der betreffenden Flächen im Elektrolytbad realisieren, wenn Frontzylinder und/oder Außenleiter bei einem eloxierbaren Metall, beispielsweise Titan oder Aluminium bestehen.

Je nach Einsatzzweck läßt sich die Elektrodenanorndung für ein Instrument flexibel ausbilden, so dass dann Innenleiter, Außenleiter, der Isolationsschlauch und ggf. auch das Isolationselement aus einem elastischen Material bestehen. Bei einem derartigen flexiblen chirurgischen Instrument läßtsich die bipolare Elektrodenanordnung unter Umständen leichter an den speziellen Behandlungsort verbringen. Alternativ lassen sich jedoch auch Innenleiter und Außenleiter starr und geradlinig ausbilden, wobei dann Frontzylinder und Außenleiter koaxial zueinander fluchtend angeordnet sind und dann durch eine geradlinige Translationsbewegung an den Behandlungsort verbracht werden können. Bei bestimmten Behandlungsorten kann es auch besonders vorteilhaft sein, das Instrument in Längsrichtung abzuwinkeln.

In allen Ausführungsformen besitzt der Außenleiter und der Frontzylinder im wesentlichen denselben Außendurchmesser, um eine behinderungsfreie Gleitbewegung der Elektrodenanordnung im Gewebe zu realisieren.

Bevorzugt bildet der Frontzylinder über seinen axialen Längenabschnitt, welcher nicht vom Isolationselement bedeckt ist, die erste Elektrode und der Außenleiter bildet über den vollen axialen Längenabschnitt, soweit dieser nicht vom Isolationselement bedeckt ist, die zweite zylindrische Elektrode. Die axiale Länge der Elektroden ist bevorzugt größer als die axiale Länge des Isolationselementes und auch größer als der Außendurchmesser des Frontzylinders und des Außenleiters. Bevorzugt beträgt die Länge des Außenleiters ein Mehrfaches der Länge des Frontzylinders. Wenn bei dieser Ausführungsform das an die Außenfläche des Instrumentes angrenzende Gewebe koaguliert ist und dadurch hochohmig wird, so kann sich bei dieser Ausführungsform der Erfindung das elektromagnetische Feld nach außen in angrenzende Gewebebereich hin verlagern, weil eine entsprechend lange zweite Elektrode vorhanden ist, so dass das elektromagnetische Feld, wenn an die Außenfläche angrenzend das Gewebe hochohmig geworden ist, radial nach außen wandern kann und dabei immer noch auf der zweiten Elektrode endet. Bei dieser Ausführungsform ist es also möglich, eine definiert in das Gewebe hineinwandernde Koagulation zu realisieren, die zum Ende kommt, wenn sich das Feld von der ersten Elektrode bis zum proximalen Ende der zweiten Elektrode erstreckt.

Umgekehrt hat sich gezeigt, dass der Beginn der Koagulation dann optimal ist, wenn die beiden Elektroden voneinander einen relativ geringen axialen Abstand voneinander besitzen, der in etwa die Größenordnung des Außendurchmessers aufweist oder nur geringfügig größer ist.

Bei einer bevorzugten Ausführungsform der Erfindung ist der Innenleiter und der Frontzylinder mit einem zentralen Hohlkanal versehen, der aus der distalen Spitze des Frontzylinders austritt und einen Lichtwellenleiter enthält, der mit sichtbarem Laserlicht beaufschlagbar ist. Auf diese Weise kann Licht an die Spitze des Applikators geleitet werden. Wenn dann beispielsweise dieser Applikator in dünnwandige Körperbereiche, beispielsweise in der Nasenmuschel zur Therapie der Concha-Hyperplasie eingesetzt wird, so ermöglicht das an der Spitze austretende Licht eine Ortung der Position der Spitze in der Nasenmuschel mit dem bloßen Auge des behandelnden Arztes. Der Arzt kann daher jederzeit erkennen, wo die Spitze der Elektrodenanordnung in der Nasenmuschei sich befindet. Das bisher beschriebene chirurgische Instrument eignet sich für einen dynamischen Einsatz, d.h. es wird beispielsweise in die vergrößerte Nasenmuschel eingestochen und bei aktivierter HF-Leistung dann aus der Nasenmuschei herausgezogen, wodurch eine schlauchförmige Koagulationszone entsteht, die sich um den Weg der Elektrodenanordnung herum gebildet hat.

Gemäß der Erfindung sind Frontzylinder, der mit dem Innenleiter in der elektrischen Verbindung steht, und der Außenleiter durch einen isolierenden Ringkörper voneinander getrennt. Bevorzugt ist der isolierende Ringkörper aus lichtdurchlässigem oder teilweise lichtdurchlässigem Material hergestellt, und in dem Ringkörper wird eine Lichtquelle angeordnet, welche ihr Licht durch den Ringkörper hindurch - bevorzugt als Streulicht - nach außen abgibt. Gemäß einer bevorzugten Ausführungsform dieses Instrumentes besitzt der Innenleiter einen Hohlkanal, der in den isolierenden Ringkörper endet und einen Lichtwellenleiter aufnimmt. Der Innenleiter ist im Bereich des Ringkörpers radial bis in den Faserkern des Lichtwellenleiters hinein mit Einschnitten versehen, so dass Licht radial an diesen Einschnitten aus dem Lichtwellenleiter austritt und durch Ringkörper hindurch diejenige Zone der bipolaren Elektrodenanordnung für den behandelnden Arzt sichtbar macht, welche zwischen den beiden Elektroden liegt, in der also die Koagulation des Gewebes jeweils erfolgt. Bei dünnwandigem Körper sieht der behandelnde Arzt also unmittelbar mit seinem Auge stets diejenige Stelle, an der koaguliert wird. Es ist dadurch eine besonders genaue lokale Behandlung des Gewebes möglich. Die Spitze des Frontzylinders läßt sich je nach Bedarf kegelförmig oder keilförmig ausbilden.

Es wird ferner beschrieben eine Elektrodenanordnung für ein chirurgisches Instrument zur elektrothermischen Koagulation von Gewebe, enthaltend einen Frontzylinder am distalen Ende des Instruments mit einer distalen Spitze, einen proximal an den Frontzylinder anschließenden länglichen, elektrisch isolierenden Träger, mindestens zwei beabstandete Elektroden auf dem Träger, die an eine Wechselspannungsquelle anschließbar sind, dadurch gekennzeichnet, dass die Elektroden streifenförmig ausgebildet sind und entlang des Trägers verlaufen.

Die Vorteile liegen insbesondere in dem einfachen Aufbau und den streifenförmig entlang des Trägers verlaufenden Elektroden. Insbesondere wenn die beiden Elektroden parallel zur Längsachse des Trägers verlaufen, eignet sich diese Elektrodenanordnung zur Therapie der Concha-Hyperplasie. Der Applikator, d.h. die Elektrodenanordnung wird dabei statisch eingesetzt, d.h. die Elektrodenanordnung wird in die vergrößerte Nasenmuschel eingestochen und verbleibt bei aktiver HF-Leistung fest in einer Position. Durch die spezielle Elektrodenkonfiguration entsteht dabei die gewünschte schlauchförmige Koagulationszone ohne dass der Applikator im Gewebe bewegt werden muss. Ein Vorteil ist weiterhin der besonders einfache Aufbau, der es ermöglicht, dass der Anschluss des HF-Generator am proximalen Ende des Trägers unmittelbar - von außen - an die Elektroden erfolgen kann.

Wenn sich die Elektroden parallel zu Längsachse des Trägers erstrecken und - beispielsweise -einander metral auf dem bevorzugt kreisförmigen Trägerquerschnitt einander gegenüberliegen, so entstehen zwei in Längsrichtung verlaufende Koagulationszonen jeweils zwischen den beiden Elektroden. Werden dagegen die Elektroden auf dem Träger längs beabstandeter Schraubenlinien aufgebracht, so wird eine entsprechend schraubenlinienförmige Zone des Gewebes behandelt und koaguliert. Bei zusätzlicher axialer Bewegung der Elektrodenanordnung entsteht dann ein kreisförmiger koagulierter Hüllkanal um den Träger herum.

Gemäß einer besonders bevorzugten Ausführungsform ist der Träger als Metallrohr ausgebildet, die eine außenliegende Isolierschicht trägt, auf welchem die streifenförmigen Elektroden aufgebracht sind. Wird als Metallrohr ein Metall verwendet, welches sich im Elektrolysebad anodisch oxidieren läßt, beispielsweise also Titan oder Aluminium, so ist die Herstellung des isolierenden Trägers besonders einfach, wenn nämlich die Außenfläche des Trägers zu einer Schicht aus Titanoxid oder Aluminiumoxid elektrolytisch eloxiert wird. Auch bei dieser Ausführungsform läßt sich durch einen Hohlkanal, der durch den Träger axial hindurchläuft an der Spitze des Frontzylinders austritt, ein Lichtwellenleiter hindurchziehen, der dem Operateur - bei entsprechend dünnwandigem Gewebe - oder bei einer Behandlung kurz unterhalb der Haut, die Position der Spitze des Frontzylinders sichtbar macht, wodurch der Operateur das Gerät zielgerichtet führen kann. Der Lichtwellenleiter läßt sich zum Beispiel mit sichtbarem Laserlicht beaufschlagen. Die distale Spitze des Frontzylinders ist vorteilhafterweise entweder kegelförmig oder keilförmig ausgebildet, und die Elektroden sind als dünne leitende Metallschichten auf den Träger aufgebracht.

Gemäß einer bevorzugten Ausführungsform läßt sich der Träger aus flexiblem Material herstellen, auf dem die streifenförmigen Elektroden sitzen. Als Träger läßt sich beispielsweise ein Lichtwellenleiter verwenden, auf dessen isolierendem Außenmantel die Elektroden streifenförmig und elastisch aufgebracht sind. Die Elektrodenanordnung läßt sich dann einfacher durch Körperöffnungen hindurch an den Behandlungsort einführen.

Auch bei dieser Ausführungsform ist die axiale Länge der Elektroden bevorzugt größer als der Außendurchmesser von Frontzylinder und -träger, die beide bevorzugt denselben Außendurchmesser aufweisen, um eine einfache, gleitende Einführung der Elektrodenanordnung in das Gewebe zu ermöglichen.

Es wird ferner eine Elektrodenanordnung beschriehen für ein chirurgisches Instrument zur elektrothermischen Koagulation im Gewebe, enthaltend einen Frontzylinder aus Metall oder Isoliermaterial am distalen Ende des Instrumentes, der Frontzylinder frontal spitz oder abgerundet ist, einen proximal an den Frontzylinder anschließenden länglichen Träger, zwei in Längsrichtung des Trägers verlaufende Elektroden, die an eine Wechselspannungsquelle anschließbar sind, dadurch gekennzeichnet, dass der Träger außen liegende, selbsttragende und in Längsrichtung verlaufende metallische Stabprofile enthält, welche mittels eines oder mehrerer isolierender Abstandselemente miteinander verbunden sind und die Elektroden bilden.

Die Vorteile ist der letztgenannten Ausführungsform liegen insbesondere darin, dass die Elektroden in Längsrichtung des Trägers verlaufen und selbsttragende metallische Stabprofile sind, die den Träger bilden, wodurch bei der Herstellung der Elektrodenanordnung Herstellungsschritte entfallen können. besonders bevorzugt ist bzw. sind die zwischen den Elektroden liegenden isolierenden Abstandselemente Lichtwellenleiter, die in Längsrichtung zwischen den Stabprofilen geführt werden und Licht an die distale Spitze des Instrumentes abgeben, damit der Operateur die Position der Instrumentenspitze jederzeit sichtbar orten kann, wenn das Instrument in dünnwandigen Körperteilen eingesetzt wird. Besonders bevorzugt lassen sich die außenliegenden, sichtbaren Lichtwellenleiter auch mit radialen Einschliffen versehen, die bewirken, dass Licht an diesen Stellen auch radial austritt. Dadurch wird dem Operateur angezeigt, über welche axiale Strecke hinweg das Instrument - bei aktivierter HF-Energie auch Gewebe koaguliert. Der Querschnitt der Stabprofile entspricht zusammengenommen bevorzugt eine Kreisfläche, alternativ kann der Querschnitt der Stabprofile auch als Umfangsabschnitt eines Rohres ausgebildet sein, wobei dann die Stabprofile beispielsweise bevorzugt einander gegenüberliegt auf dem Außenmantel eines Lichtwellenleiters befestigt sind und auf diese Weise einen starren Träger mit außenliegenden, in Längsrichtung streifenförmigen Elektroden bilden.

Weiter wird beschrieben eine Elektrodenanordnung für ein chirurgisches Instrument zur elektrothermischen Koagulation im Gewebe, enthaltend einen Frontzylinder aus Metall oder Isoliermaterial am distalen Ende des Instrumentes, wobei der Frontzylinder frontal spitz oder abgerundet ist, einen proximal an den Frontzylinder anschließenden länglichen Träger, zwei in Längsrichtung des Trägers verlaufende Elektroden, die an eine Wechselspannungsquelle anschließbar sind, dadurch gekennzeichnet, dass die Elektroden (2, 4) zylinderförmige Rohrabschnitte (82, 84) aus Metall sind, welche in vorgegebenem Abstand voneinander in Längsrichtung mit dem Träger axial fluchtend angeordnet sind.

Gemäß dieser bevorzugten Ausführungsform ist die erste Elektrode als selbsttragender Rohrabschnitt ausbildbar, der zwischen dem Frontzylinder und einem isolierenden rohrförmigen ersten Träger sitzt, und die zweite Elektrode ist ebenfalls als selbsttragender Rohrabschnitt ausbildbar, der zwischen dem ersten Träger und einem zweiten rohrförmigen Träger angeordnet ist, wobei die Endabschnitte der Elektroden auf dem Frontzylinder, dem ersten und dem zweiten Träger über einen vorgegebenen Längenabschnitt aufliegt. Alternativ ist es auch möglich, dass die zweite Elektrode sich bis zum proximalen Endabschnitt erstreckt. Besonders bevorzugt sind bei dieser Ausführungsform die Längenabschnitte der Elektroden, die auf dem ersten und/oder dem zweiten Träger aufliegen, mit einer Isolierschicht bedeckt sind. Außerdem ist innerhalb des Hohlkanals ein Spülschlauch vorgesehen, der vom proximalen Ende des Instrumentes bis zum Frontzylinder, also auch durch die Rohrabschnitte, welche die Elektroden bilden, hindurchläuft um sich bis zum Frontzylinder hin erstreckt und Flüssigkeit am distalen Ende in den Hohlkanal abgibt, in welchem die Flüssigkeit - in Kontakt mit den Elektroden - zum proximalen Ende des Instrumentes zurückströmt.

Durch die Kühlung der Elektrodenflächen mittels einer Spülflüssigkeit wird der sogenannte "Hot-Spot" der Koagulation etwa zwei bis drei Millimeter von der Instrumentenoberfläche in das Gewebe hineinverlagert. Durch die Kühlung wird gewährleistet, dass die Gewebe-Elektroden-Kontaktfläche immer unter einer vorgegebenen Temperatur gehalten wird und daher nicht so stark austrocknet, so dass auch der Energieeintrag in das angrenzende Gewebe über einen längeren Zeitraum gewährleistet ist. Ganz besonders vorteilhaft ist es dabei, dass die Längenabschnitte der Elektroden, die auf dem isolierenden Trägern - oder in einer speziellen Ausführungsform auch auf den als Isolierkörper ausgebildeten Frontzylinder - aufliegen und daher von der Kühlflüssigkeit nicht direkt gekühlt werden, mit einer Isolierschicht bedeckt sind. Dadurch werden diese weniger gekühlten Längenabschnitte, die sich folglich stärker erhitzen als die gekühlten Elektrodenabschnitte, von dem Isolator abgedeckt und kommen deshalb mit dem angrenzenden Gewebe nur über die vergleichsweise kühlere Isolatorschicht in Kontakt. Die Abdeckung der von der Spülflüssigkeit nicht gekühlten Endabschnitte der Elektroden durch Insolierschichten hat somit zur Folge, dass das angrenzende Gewebe auch in diesen Längenabschnitten nicht zu heiß wird und damit austrocknet.

Bei einer bevorzugten Ausführungsform wird ein selbsttragendes Metallrohr zwischen dem Frontzylinder und dem Träger vorgesehen. Ein distaler Rohrabschnitt des Metallrohres dient als erste Elektrode, ein daran angrenzender proximaler Rohrabschnitt ist mit einer zylindrischen Isolierschicht umgeben und trägt auf dieser Isolierschicht eine Metallschicht, welche als zweite Elektrode dient. Alternativ lässt sich auch der proximale Rohrabschnitt als zweite Elektrode heranziehen, und auf dem distalen Rohrabschnitt wird dann eine zylindrische Isolierschicht aufgebracht, die mit einer Metallschicht beschichtet wird, wobei diese distale Metallschicht dann als erste Elektrode dient.

Diese Ausführungsform besitzt den Vorteil, dass eine einfache Herstellbarkeit und Montage der bipolaren Elektrodenanordnung gegeben ist. Dies gilt insbesondere dann, wenn als Metall ein eloxierbares Material, beispielsweise Titan oder Aluminium verwendet wird, und die auf dem Rohrabschnitt aufgebrachte zylindrische Isolierschicht durch anodische Oxidation (eloxieren) der Metalloberfläche erzeugt wird, wobei die darauf abgelegte Metallschicht beispielsweise durch Aufdampfen oder elektrolytische Beschichtung erzeugt werden kann. Bei dieser Ausführungsform weist der Träger einen Hohlkanal auf, der sich in einen Hohlkanal durch das metallische Rohr fortsetzt. Die Anschlussleitungen für die Elektroden verlaufen von den Elektroden durch den Hohlkanal bis zum proximalen Ende des Instrumentes. Es ist möglich einen Spülschlauch durch den Hohlkanal hindurch zuführen, der sich bis zum Frontzylinder erstreckt und Kühlflüssigkeit am distalen Ende in den Hohlkanal abgibt.

Vorteilhafterweise ist in Verlängerung des Hohlkanals des Trägers im Frontzylinder eine Ausnehmung vorgesehen, in der sich ein Temperatursensor befindet, dessen Anschlussleitung durch den Hohlkanal zum proximalen Ende des Instrumentes geführt ist. Dadurch es möglich - beispielsweise zur Therapie der benignen Prostatahyperptasie einen Temperatursensor oder Thermistor in die Spitze des Frontzylinders zu setzen, der zur Messung der Gewebetemperatur eingesetzt werden kann. Bevorzugt ist der Träger aus einem flexiblen isolierenden Schlauch oder Rohr gebildet, und die Elektroden sind zylinderförmige selbsttragende Rohrabschnitte aus Metall, die in vorgegebenem Abstand auf dem Träger befestigt sind. Auch bei dieser Ausführungsform der Erfindung entspricht der Außendurchmesser des Frontzylinders dem Außendurchmesser der Elektroden und die axiale Länge der Elektroden ist größer als der Durchmesser, der axiale Abstand der beiden Elektroden voneinander ist etwa gleich oder kleiner als ihr Außendurchmesser. Es hat sich gezeigt, dass bei dieser Dimensionierung das elektrische Feld, welches die Koagulation des Gewebes bewirkt, ausreichend stark erzeugt werden kann und auch - nachdem das an die Außenfläche angrenzende Gewebe koaguliert ist - ausreichend weit sich in das Gewebe hinein ausbreiten kann, so dass eine vorteilhafte große Koagulationszone realisiert wird.

Der Temperatursensor in der Ausnehmung des Frontzylinders ist bevorzugt in Kunstharz oder in ein Kleberbett eingebettet, welches die Temperatur des metallischen Frontzylinders gut leitend an den Temperatursensor abgibt. Der Außendurchmesser der Elektroden und der Außendurchmesser des Frontzylinders sind identisch, und der Zwischenraum zwischen den Elektroden wird von Isoliermaterial ausgefüllt, damit auch dieser Längenabschnitt den ansonsten vorliegenden Außendurchmesser aufweist. Dadurch ist ein uniformer Querschnitt über der distalen Spitze bis zum proximalen Ende der zweiten Elektrode realisiert, wogegen der flexible Träger im Anschluss hieran einen reduzierteren Außendurchmesser aufweisen kann. Die Konstanz die Außendurchmessers im Bereich des Frontzylinders und der Elektroden hat zur Folge, dass sich das Instrument leicht, insbesondere ohne Hindernisse, in das Gewebe einführen läßt.

Ferner wird beschrieben eine Elektrodenanordnung für ein chirurgisches Instrument zur elektrothermischen Koagulation von Gewebe, enthaltend einen Frontzylinder aus Metall am distalen Ende des Instruments, einen proximal an den Zylinder anschließenden länglichen, zylindrischen Träger aus Isoliermaterial, zwei beabstandete zylindrische Elektroden, dadurch gekennzeichnet, dass der metallische Frontzylinder am distalen Ende abgerundet und im Anschluss an den runden Endabschnitt einen Zylinderabschnitt vorgegebener Länge aufweist, dass der Frontzylinder die erste Elektrode bildet, und dass auf dem Träger in vorgegebenem axialen Abstand von dem Frontzylinder eine Metallschicht als zweite Elektrode aufgebracht ist.

Durch den Träger verläuft in dieser bevorzugten Ausführungsform ein Hohlkanal, der sich bis in den Frontzylinder hinein fortsetzt und einen Spülschlauch aufnimmt, der an seinem distalen Ende Spülflüssigkeit abgibt, die an der Innenwand des Frontzylinders entlang und schließlich zwischen Träger und dem Spülschlauch zum proximalen Ende zurückströmt.

Der Frontzylinder ist mit seinem proximalen Ende in einer ringförmigen Ausnehmung des Trägers befestigbar und der Überlappungsbereich zwischen Träger und Frontzylinder weist außen eine Oxidschicht auf dem metallischen Frontzylinder auf, die verhindert, dass das an dieser Stelle ungekühlte Metall des Frontzylinders überhitzt mit dem angrenzenden Gewebe in Kontakt kommt.

Der Träger lässt sich sowohl flexibel als auch starr ausbilden, und da der Frontzylinder aus metallischem Material gebildet ist, ermöglicht es dieses Instrument - aufgrund der aktiven Spitze - Randtumore zu behandeln, die unmittelbar vor dem abgerundeten Frontzylinder liegen. Voraussetzung für eine vorteilhafte Funktion dieser Elektrodenanordnung ist es, dass die Wärmeabfuhr durch den Kühlkreislauf bei beiden Elektroden möglichst gleich ist. Dies wird erreicht, wenn die mittlere Stromdichte auf der ersten, d.h. distalen Elektrode größer oder gleich der Stromdichte auf der zweiten, proximalen Elektrode ist. Diese Bedingung ist dann erfüllt, wenn die Oberfläche A₁ der ersten Elektrode kleiner oder gleich der Oberfläche A₂ der zweiten Elektrode ist. Sofern auch der Flächenanteil des runden Endabschnittes - mit seinem Radius R - berücksichtigt wird, so ergibt sich für die Längen L₁, L₂ der ersten und zweiten Elektrode folgendes Verhältnis: L₁ + R ≤ L₂. Wenn sehr große Längen L₂ der zweiten Elektrode realisiert werden sollen und trotzdem die Flexibilität des Applikators erhalten bleiben soll, kann die zweite Elektrode durch Beschichten des flexiblen Trägers mit einer Metallschicht aufgebaut werden.

Weiter wird ebenfalls beschrieben eine Elektrodenanordnung für ein chirurgisches Instrument zur elektrothermischen Koagulation von Gewebe enthaltend: einen länglichen, zylindrischen Träger aus einem Metallrohr oder Metallstab, zwei beabstandete zylindrische Elektroden auf dem Träger, dadurch gekennzeichnet, dass die erste Elektrode ein distaler Abschnitt des Trägers ist, dass auf einem axial daran angrenzenden Abschnitt eine Isolierschicht auf den Träger aufgebracht ist, und dass auf der Isolierschicht in vorgegebenem axialen Abstand von der ersten Elektrode eine zylindrische Metallschicht als zweite Elektrode angeordnet ist.

Bei dieser Ausführungsform wird der Frontzylinder und der Träger als einstückiges Metallrohr oder Metallstab verwirklicht, dessen distales Ende angespitzt ist. Ein distaler Abschnitt des Metallrohres oder Metallstabes bildet die erste Elektrode. Daran angrenzend wird eine Isolierschicht auf den Träger aufgebracht, und im proximalen Bereich dieser Isolierschicht wird dann eine zylindrische Metallschicht auf die Isolierschicht abgelegt und bildet die zweite, zylindrische Elektrode. Die Isolierschicht lässt sich durch einen Kunststoffschlauch verwirklichen, auf den - als zweite Elektrode -eine Metallbeschichtung aufgebracht ist. Der metallische Träger mit-distaler Spitze stellt eine bipolare Elektrodenanordnung in Form einer Kanüle oder Nadel dar und eignet sich besonders zur Therapie von erweiterten Endgefäßen wie z.B. Besenreiservarizen. Die Elektrodenanordnung wird mit ihrer Spitze in Längsrichtung in das erweiterte Gefäß eingestochen. Bei Aktivierung der HF-Leistung koaguliert das Blut und die Gefäßwand primär um die erste Elektrode. Dabei zieht sich das Gefäß zusammen, so dass ein Verschluss erzielt wird, mit der Folge, dass dann kein Blut mehr in das Gefäß fließen kann, wodurch dieses nicht mehr durch die Haut zu erkennen ist und der gewünschte kosmetische Effekt erzielt wird.

Besonders vorteilhaft werden Isoiatorschichten, die bei den bipolaren Elektrodenanordnungen Verwendung finden, aus Keramikmaterial eingesetzt. Der Vorteil dieses Materials ist, dass es eine hohe mechanische Festigkeit bietet und mittels einer elektrolytischen Anodisierung (Eloxierung) beispielsweise auf Titan in Form von Titanoxid, oder bei Aluminium in Form von Aluminiumoxid in einfacher Weise erzeugen lässt. Die Schichtdicke hängt ab von der bei der Elektrolyse eingesetzten elektrischen Spannung. Statt Titan eignen sich auch verschiedene Titanlegierungen als Ausgangsmaterial, auf den durch anodisches Oxidieren die Keramikschicht erzeugt wird. Um eine derartige vollständige oder partielle Beschichtung von Titan oder geeigneten Titanlegierungen oder Aluminium vorzunehmen, wird zuerst der entsprechende Metallkörper chemisch vorgereinigt, um fettfreie und oxidfreie Oberflächen zu erhalten. Im Anschluss daran werden die nicht zu beschichtenden Stellen maskiert. Die Maskierung kann durch spezielle Lacke oder Schichten aber auch durch Schrumpfschläuche verwirklicht werden. Zur anodischen Aufbringung einer keramischen Schicht ist das Ausgangsmaterial, also Titan, Titanlegierungen oder Aluminium, elektrisch zu kontaktieren und als Anode mit Spannung zu beaufschlagen.

Um beispielsweise - auf der Basis von Titan als Ausgangmaterial - eine Titanoxid-Keramikschicht aufzubringen, sind folgende Maßnahmen zu treffen: Um das Titan an seiner Oberfläche in seine Ionenphase zu überführen, ist eine entsprechend molare Säure in wässriger Lösung einzusetzen. Die in Frage kommenden molaren Lösungen liegen zwischen 0,1 bis 1 Molar H₂So₄ (Schwefelsäure) bzw. H₃Po₄ (Phosphorsäure). Durch anlegen einer entsprechenden Gleichspannung scheidet sich an der Anode, der hier zu beschichtenden Titanelektrode, Sauerstoff ab und verbindet sich mit der ionisierten Titanoberfläche und bildet sich zu Titanoxid um. Die einzusetzenden Gleichspannungen und Ströme liegen je nach Schichtdicke zwischen 10V und 500V bei maximalen Strömen von 1 A. Dadurch durchläuft der Oxidationsprozess mehrere Oxidationsstufen (Titanoxide) je nach Länge des Prozesses. Die mit diesen Verfahren zu erzielenden Schichtdicken liegen in der Größenordnung von 20 bis 30 µm. Mit Hilfe der über die Interferenzfarben darzustellenden Schichtdicken wird durch die unterschiedliche Lichtbrechung an der Grenzfläche zum Metall (die Oxidschicht ist transparent) diese proportional über ein Farbspektrum darstellbar. Mit dieser Methode lassen sich spezielle paramagnetische Elektroden aus Titan - oder Titanlegierungen wie TiAl₆V₄ - effizient mit einer dielektrischen Keramikschicht variabel hinsichtlich Schichtdicke und/oder Farbe versehen.

Neben den guten dielektrischen Eigenschaften der auf diese Weise erzeugten Keramikschichten sind auch die tribologischen Eigenschaften hevorragend geeignet, um ggf. Abriebfestigkeiten und Oberflächengüte zu erhöhen. Diese farbigen Keramikschichten eignen sich auch zur stabilen Markierung von Nadeln, Kanülen oder Sonden. Durch die Wahl der Schichtdicke ist eine proportionale Interferenzfarbe zu wählen. Damit können Farben von Grau, Gold, Violett bis hin zu Blau eingestellt werden.

Gemäß einer weiteren bevorzugten Ausführungsform besitzt mindestens eine der Anschlussleitungen, die zum Anschließen der Elektroden dienen, an ihrem Ende einen Abschnitt aus Federmetall, bevorzugt Federdraht, der eine solche Formgebung aufweist, dass er sich im Hohlkanal - innerhalb der Elektroden - radial nach außen gegen die Innenfläche der Elektroden verklemmt und dadurch den elektrischen Kontakt ausreichend sicher und zuverlässig herstellt. Der Federmetallabschnitt der so ausgebildeten Anschlussleitungen ist bevorzugterweise zu einer Spiralfeder gewickelt, die mit einer vorgegebenen Zugspannung im Spiraldraht beaufschlagt ist, deren Wickel unter dieser Zugspannung also einen reduzierten Durchmesser aufweist, um sich von außen in einfacher Weise in den Hohlraum der Elektroden einführen zu lassen. Anschließend wird die auf den Federdraht wirkende Zugvorspannung beseitigt, die Spiralfeder erreicht dann ihren vollen Außendurchmesser und legt sich dabei von innen gegen die Innenflächen der Elektroden selbstklemmend an. Um die Einführung der entsprechenden Federabschnitte am Ende der Anschlussleitungen in einfacher Weise durchführen zu können, ist ein entsprechendes Spezialwerkzeug einsetzbar, welches es gestattet, die Spiralfeder mit reduziertem Durchmesser einzusetzen, dann die Draht-Zugvorspannung zu beseitigen und damit die Spiralfeder gegen die Innenfläche der Elektrode zur Anlage zu bringen.

Vorteilhafte Weiterbildungen der Erfindung sind durch die Merkmale der Unteransprüche gekennzeichnet.

Im folgenden werden Ausführungsbeispiele der Erfindung anhand der Zeichnung näher erläutert. Es zeigen:
- Fig. 1: einen Längsschnitt durch eine erste Ausführungsform einer bipolaren Elektrodenanordnung für ein chirurgisches Instrument;
- Fig. 2: einen Längsschnitt einer zweiten Ausführungsform einer bipolaren Elektrodenanordnung;
- Fig. 3: einen Längsschnitt einer dritten Ausführungsform einer bipolaren Elektrodenanordnung;
- Fig. 4: einen Längsschnitt einer vierten Ausführungsform einer bipolaren Elektrodenanordnung;
- Fig. 5: einen Längsschnitt durch eine fünfte Ausführungsform der bipolaren Elektrodenanordnung;
- Fig. 6: einen Längsschnitt durch eine sechste Ausführungsform der bipolaren Elektrodenanordnung;
- Fig. 7: einen Längsschnitt durch eine siebte Ausführungsform der bipolaren Elektrodenanordnung;
- Fig. 8: einen Querschnitt durch die siebte Ausführungsform;
- Fig. 9: einen Längsschnitt durch eine achte Ausführungsform der bipolaren Elektrodenanordnung;
- Fig. 10: einen Querschnitt durch die achte Ausführungsform;
- Fig. 11: einen Längsschnitt durch eine neunte Ausführungsform der bipolaren Elektrodenanordnung;
- Fig. 12: einen Querschnitt durch die neunte Ausführungsform;
- Fig. 13: einen Querschnitt durch eine zehnte Ausführungsform der bipolaren Elektrodenanordnung;
- Fig. 14: einen Querschnitt durch eine elfte Ausführungsform der bipolaren Elektrodenanordnung;
- Fig. 15: eine Seitenansicht einer zwölften Ausführungsform der bipolaren Elektrodenanordnung;
- Fig. 16: einen Längsschnitt durch die zwölfte Ausführungsform;
- Fig. 17: einen Längsschnitt durch eine dreizehnte Ausführungsform der bipolaren Elektrodenanordnung mit einem spitzen Frontzylinder aus Isoliermaterial;
- Fig. 18: einen Längsschnitt durch die 13. Ausführungsform mit einem metallischen spitzen Frontzylinder;
- Fig. 19: einen Längsschnitt durch die 13. Ausführungsform mit einem metallischen, frontal abgerundeten Frontzylinder;
- Fig. 20: einen Längsschnitt durch eine vierzehnte Ausführungsform der bipolaren Elektrodenanordnung;
- Fig. 21: einen Längsschnitt, durch eine fünfzehnte Ausführungsform der bipolaren Elektrodenanordnung;
- Fig. 22: einen Längsschnitt durch eine sechzehnte Ausführungsform der bipolaren Elektrodenanordnung;
- Fig. 23: einen Längsschnitt durch eine siebzehnte Ausführungsform der bipolaren Elektrodenanordnung;
- Fig. 24: einen Längsschnitt durch eine achtzehnte Ausführungsform der bipolaren Elektrodenanordnung;
- Fig. 25: einen Längsschnitt durch eine Variante der 18. Ausführungsform;
- Fig. 26: einen Längsschnitt durch eine zweite Variante der 18. Ausführungsform;
- Fig. 27: eine perspektivische Ansicht eines Frontzylinders mit einer Teilbeschichtung mit Keramikmaterial;
- Fig. 28: einen Endabschnitt einer Anschlussleitung zum Anschließen einer Elektrode; und
- Fig. 29: eine perspektivische Ansicht einer aus einem Metallrohr gebildeten Elektrode mit einer Keramikbeschichtung und einer im Inneren des Robres angebrachten Anschlussleitung.

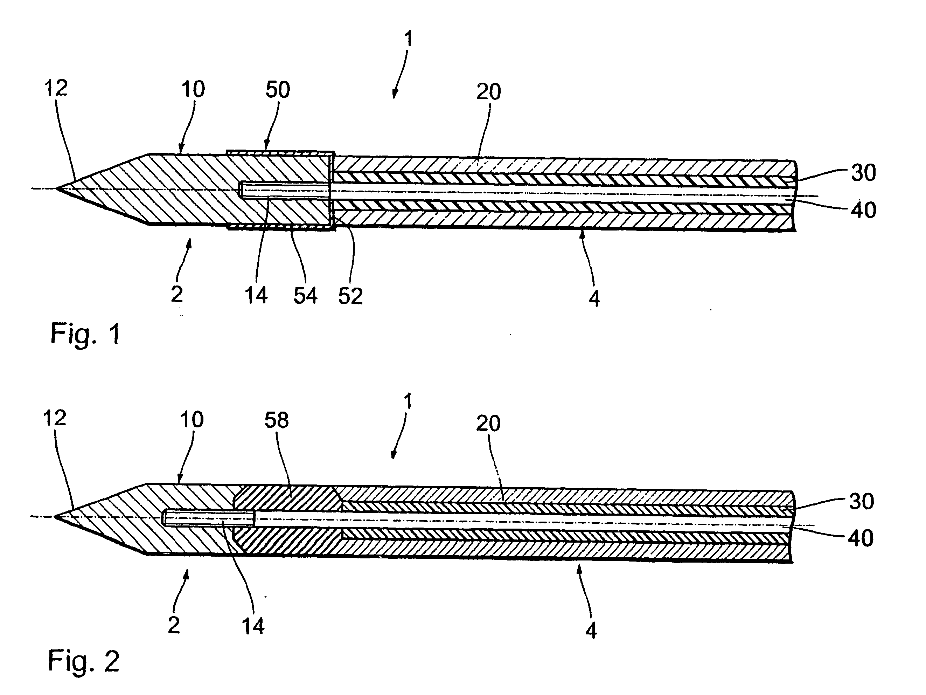

Fig. 1 zeigt einen Längsschnitt durch eine erste, jedoch nicht erfindungsgemäße, Ausführungsform einer bipolaren Elektrodenanordnung, die Bestandteil eines chirurgischen Instrumentes zur elektrothermischen Koagulation von Gewebe ist. Die Elektrodenanordnung enthält einen elektrisch leitenden Frontzylinder 10, der das distale, d.h. vom Benutzer des Instruments abgewandte Ende des Instruments bildet. Der Frontzylinder endet an seinem freien Ende in einer Spitze 12, die in der dargestellten Ausführungsform kegelförmig spitz ausläuft. An den Frontzylinder 10 schließt ein rohrförmiger Außenleiter 20 an, der in seinem Innenraum einen Isolationsschlauch 30 aufnimmt, durch den ein stabförmiger Innenleiter 40 hindurchverläuft. Der stabförmige Innenleiter 40 besitzt an seinem distalen Ende ein Außengewinde, welches in ein entsprechendes in axialer Längsrichtung verlaufendes Innengewinde verschraubbar ist und mittels dieser Schraubverbindung 14 elektrisch und mechanisch mit dem Frontzylinder 10 verbunden ist.

Zwischen dem Frontzylinder 10 und dem Außenleiter 20 ist ein Isolatorelement 50 angeordnet, welches eine radiale Trennwand 52 zwischen Frontzylinder 10 und der distalen Stirnwand des Außenleiters 20 und des Isolationsschlauches 30 besitzt. Außen an der Trennwand 52 geht das Isolatorelement 50 in eine Hüllwand 54 über, die - in der dargestellten Ausführungsform - die Außenfläche des Frontzylinders 10 enganliegend umgibt, die jedoch in einer alternativen Ausführungsform auch - nach proximal gerichtet - die Außenfläche des Außenleiters 20 umgeben kann. Die freiliegende Außenfläche des Frontzylinders 10 bildet eine erste Elektrode 2. Die freiliegende Außenfläche des Außenleiters 20 bildet eine zweite Elektrode 4. An die beiden Elektroden wird - am proximalen Ende der Elektrodenanordnung - eine Hochfrequenz-Wechselspannungsquelle angeschlossen, wenn die bipolare Elektrodenanordnung in das zu behandelnde menschliche oder tierische Gewebe eingeführt ist und das Gewebe durch Wärmewirkung des elektrischen Feldes koaguliert werden soll.

Fig. 2 zeigt eine zweite erfindungsgemäße Ausführungsform der bipolaren Elektrodenanordnung, bei der wiederum ein Frontzylinder 10 in eine kegelförmige distale Spitze 12 ausläuft, wobei wiederum ein stabförmiger metallischer Innenleiter von einem Isolationsschlauch 30 umgeben ist, der seinerseits von einem metallischen rohrförmigen Außenleiter 20 umgeben wird. Zwischen dem Frontzylinder 10 und der Stirnwand des Außenleiters und des Isolationsschlauches 30 ist ein Isolatorelement 50 vorgesehen, welches die Form eines Ringkörpers 58 besitzt und eine vorgegebene axiale Länge aufweist, die den Frontzylinder 10 und den Außenleiter 20 auf Abstand hält. Der Frontzylinder 10 besteht aus Metall und dient als zylindrische erste Elektrode 2. Der Außenleiter 20 ist ebenfalls aus Metall hergestellt und dient als zylindrische zweite Elektrode 4. Der Frontzylinder 10 ist mittels einer Schraubverbindung 14 mit dem Innenleiter 40 verbunden. Am proximalen Ende der Elektrodenanordnung wird zwischen dem Außenleiter 20 und dem Innenleiter 40 eine HF-Wechselspannungsquelle angeschlossen, wenn eine elektrothermische Koagulation von umgebendem Gewebe durchgeführt werden soll.

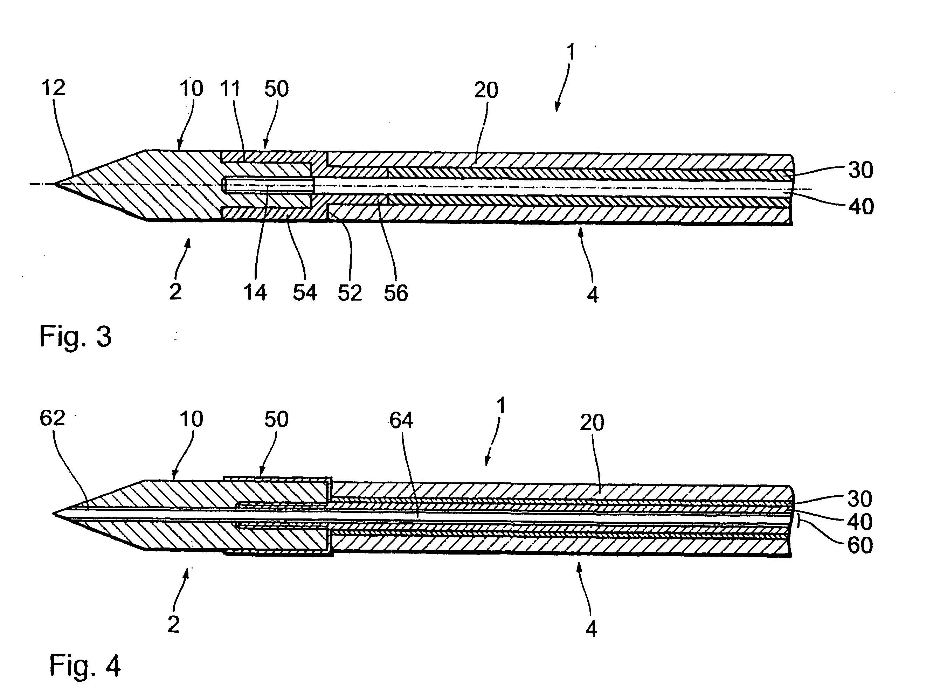

Fig. 3 zeigt eine den Fig. 1 und 2 entsprechende Ausführungsform einer bipolaren Elektrodenanordnung, bei der das Isolatorelement 50 becherförmig ausgebildet ist, wobei die zylindrische Hüllwand 54 in einer entsprechenden Ringausnehmung 11 des Frontzylinders 10 sitzt. Außerdem wird die radiale Trennwand 52 des Isolatorelements 50 nach proximal schlauchförmig - mit dem Außendurchmesser des Isolationsschlauches 30 - gegen den Isolationsschlauch 30 geführt, der eine entsprechende axiale Länge vor der distalen Stirnwand des Außenleiters 20 endet.

Fig. 4 zeigt eine vierte Ausführungsform einer bipolaren Elektrodenanordnung 1, die weitgehend der Anordnung gemäß Fig. 1 entspricht, wobei gleiche Teile mit denselben Bezugszeichen versehen sind. Zusätzlich zu der Anordnung gemäß Fig. 1 verläuft zentral durch den stabförmigen Innenleiter 14 und hierzu fluchtend auch durch den Frontzylinder 10 ein Hohlkanal, durch den ein Lichtwellenleiter hindurchläuft, der sichtbares Licht an die distale Spitze 12 der Elektrodenanordnung führt, wenn der Lichtwellenleiter proximal beispielsweise mit sichtbarem Laserlicht gespeist wird. Der Lichtwellenleiter 60 enthält einen Mantel 62, der den lichtleitenden Kern 64 umgibt. Um den Mantel herum kann auch noch eine Umhüllung (Cladding) vorgesehen werden.

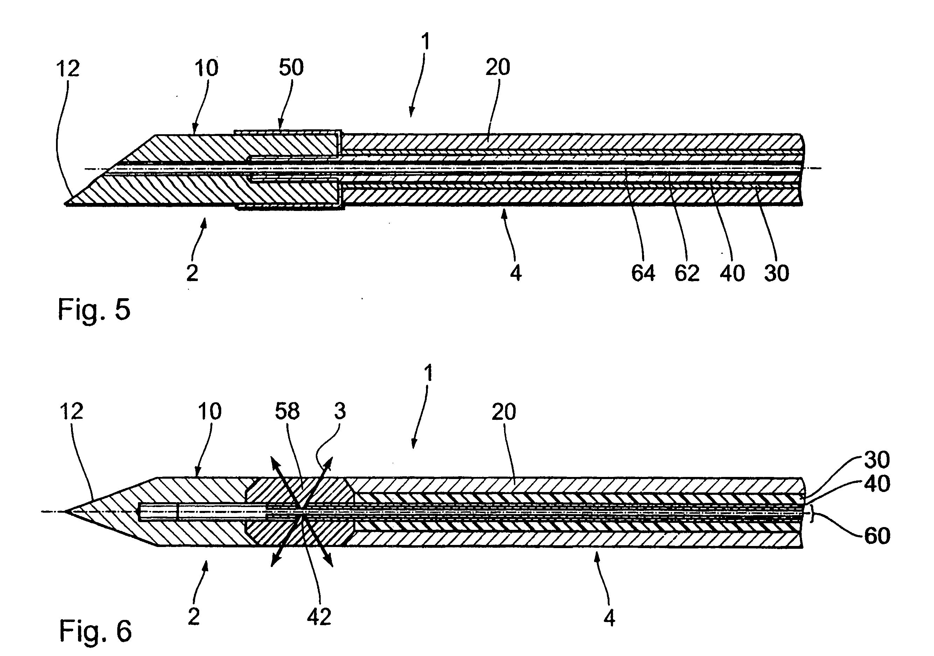

Fig. 5 zeigt eine weitere Ausführungsform die weitgehend der Ausführungsform gemäß Fig. 4 entspricht, wobei jedoch der Frontzylinder 10 an seinem distalen Ende eine keilförmige Spitze 12 besitzt. Wiederum ist durch den Innenleiter 40 und hieran anschließend auch durch den Frontzylinder 10 ein zentraler Hohlkanal vorhanden, durch den ein Lichtwellenleiter 60 mit seinem Mantel 62 und dem Kern 64 bis zur distalen Spitze 12 hindurchläuft und dem Benutzer der Elektrodenanordnung - insbesondere bei einer Behandlung von Gewebe in dünnwandigen Körperteilen - jeweils optisch die Position der distalen Spitze 12 im Gewebe anzeigt.

Fig. 6 zeigt eine weitere Ausführungsform der erfindungsgemäßen bipolaren Elekrodenanordnung 1, die im wesentlichen der Ausführungsform gemäß Fig. 2 oder 4 entspricht. Zwischen dem Frontzylinder 10 und der konzentrischen Anordnung aus Außenleiter 20, Isolationsstoff 30 und Innenleiter 40 ist ein Ringkörper 58 vorgesehen, durch den der Innenleiter 40 bis zum Frontzylinder 10 axial hindurchläuft. Der Innenleiter 40 weist einen zentralen Hohlkanal auf, der sich bis zum distalen Ende des Ringkörpers 58 hin erstreckt und einen Lichtwellenleiter 60 enthält. Der Ringkörper 58 ist aus transparentem oder halbtransparentem Material ausgebildet und läßt Licht nach außen hindurchtreten. Im Bereich des Ringkörpers 58 sind in den Innenleiter bis hinein in den Kern 64 des Lichtwellenleiters hinein radiale Einschliffe 42 eingebracht, mit der Folge, dass durch diese Einschliffe Licht radial aus dem Innenleiter 40 und durch den Ringkörper 58 hindurch nach außen tritt, so dass der Operateur die Position des elektrischen Feldes, welches sich zwischen der ersten Elektrode 2 und der zweiten Elektrode 4 ausbildet, wenn an den Innenleiter 40 und den Außenleiter 20 HF-Leistung eingespeist wird, optisch sichtbar gemacht werden kann. Bevorzugt ist der Ringkörper 48 aus einem solchen Material bzw. seine Oberfläche weist eine solche Struktur auf, dass das aus dem Lichtwellenleiter austretende Licht 3 als Streulicht nach außen tritt.

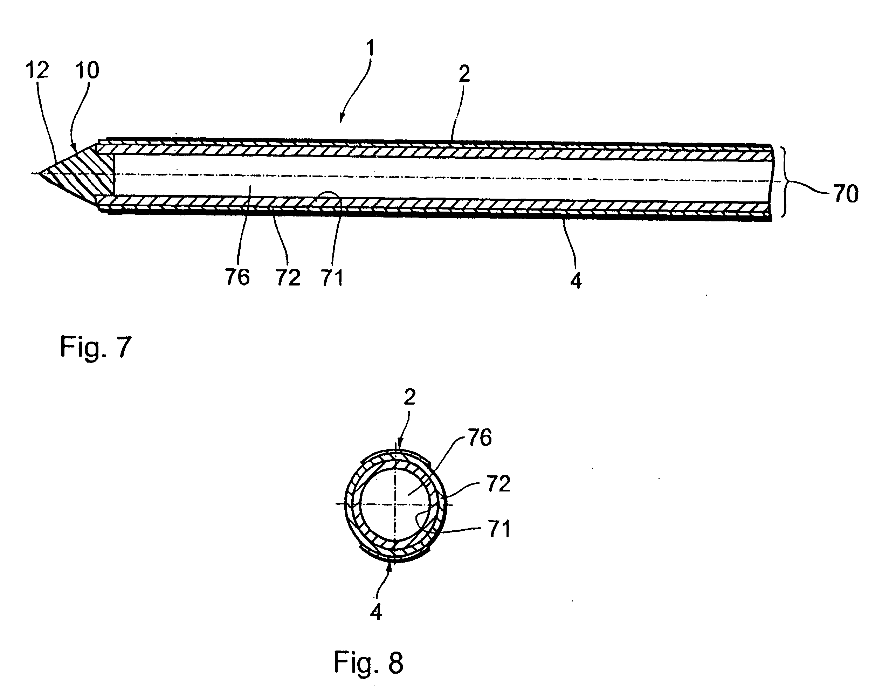

In den Fig. 7 und 8 ist eine weitere Ausführungsform der bipolaren Elektrodenanordnung 1 im Längsschnitt und im Querschnitt dargestellt. An einen Frontzylinder 10, der eine distale Spitze 12 aufweist, schließt proximal ein länglicher, elektrisch isolierender Träger 70 an, der - in der dargestellten Ausführungsform - als ein Metallrohr 71 mit einer außen liegenden Isolierschicht 72 ausgebildet ist. Beispielsweise besteht das Metallrohr aus Titan oder einer Titanlegierung, und die Isolationsschicht 72 wird durch Eloxieren der Oberfläche des Rohres 71 als dünne Keramikschicht hergestellt. Auf der Isolationsschicht 72 sind zwei streifenförmige Elektroden 2, 4 aufgebracht, die sich parallel zueinander in Längsrichtung des Trägers 70 erstrecken und - auf dem Umfang der Isolierschicht, vgl. Fig. 8 - einander diametral gegenüberliegen. Bei dieser Elektrodenanordnung wird ein elektrisches Feld in Längsrichtung des Trägers, nämlich längs der gesamten streifenförmigen Elektrode 2, 4 ausgeprägt, so dass auch entsprechende Koagulationsstreifen in Längsrichtung der bipolaren Elektrodenanordnung erzeugt werden, wenn an die Elektroden 2, 4 eine entsprechende HF-Wechselspannungsquelle angelegt ist.

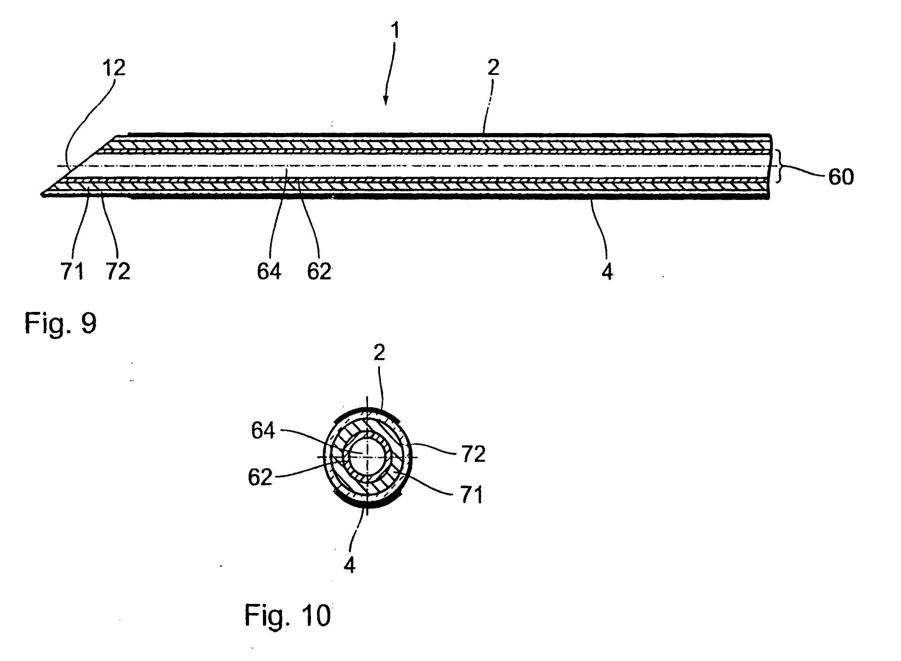

Die Fig. 9 und 10 zeigen eine weitere Ausführungsform einer bipolaren Elektrodenanordnung 1 für ein chirurgisches Instrument, wobei diese Ausführungsform eine Fortentwicklung der Ausführungsform gemäß den Fig. 7 und 8 darstellt. Ein länglicher, uniformer, isolierender Träger 70 besteht aus einem metallischen Rohr 71, das vollständig mit einer Isolationsschicht 72 beschichtet ist und durchgängig bis zur distalen Spitze 12 hin einen zentralen Hohlkanal aufweist, durch den ein Lichtwellenleiter 60 bis zur distalen Spitze 12 hindurchläuft. Der Lichtwellenleiter 60 besteht aus einem lichtleitenden Kern 64 und einem diesen Kern umgebenden Mantel 62. Außen auf der Isolationsschicht 72 sind in Längsrichtung, also zueinander parallel, zwei streifenförmige Elektroden 2, 4 aufgebracht, die sich über die gesamte dargestellte Länge des Trägers 70 hinwegerstrecken und auf der Isolationsschicht 72 befestigt sind.

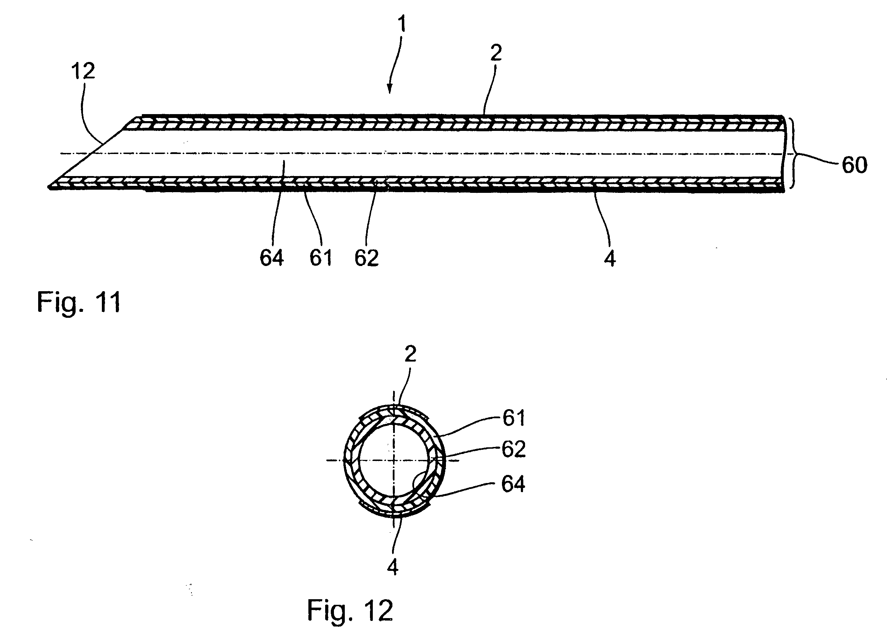

Die Fig. 11 und 12 zeigen eine weitere Ausführungsform, die weitgehend der Ausführungsform gemäß den Fig. 9 und 10 entspricht, wobei jedoch kein rohrförmiger metallischer Träger 70 zum Einsatz gelangt. Die Fig. 11 und 12 zeigen vielmehr eine Ausführungsform, bei der ein Lichtwellenleiter 60 mit einem Kern 64, einem Mantel 62 und einer Kunststoff-Ummantelung (Cladding) 61 an seinem freien Ende mit einer keilförmigen Spitze 12 versehen wird und auf dem Mantel oder dem Cladding 61 in Längsrichtung 2 streifenförmige Elektroden 2, 4 aufweist, die am Umfang einander gegenüberliegend angeordnet sind. Die Elektroden 2, 4 sind in dieser Ausführungsform als flexible Schichten aufgebracht, so dass die gesamte bipolare Elektrodenanordnung flexibel ist.

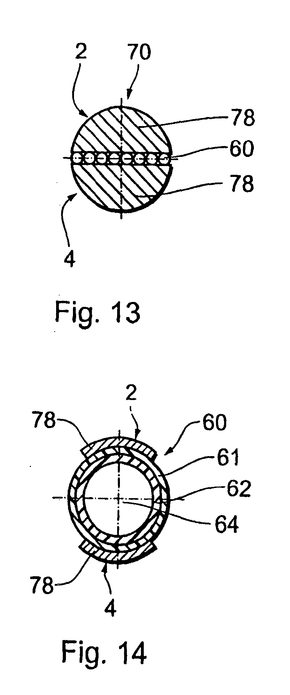

In den Fig. 13 und 14 sind Querschnitte von bipolaren Elektrodenanordnungen gezeigt, bei denen der Träger 70 aus selbsttragenden, in Längsrichtung verlaufenden metallischen Stabprofilen 76 gebildet ist. In Fig. 13 sind zwei Stabprofile 76 aus Metall von Abstandselementen 60 isolierend beabstandet. Die Stabprofile 76 besitzen jeweils etwa einen Querschnitt einer Halbkreisfläche und bilden längliche, in Längsrichtung der Elektrodenanordnung verlaufende Elektroden 2, 4. Die Abstandselemente 60 sind in der dargestellten Ausführungsform als Lichtleiter 60 ausgebildet. Bevorzugt werden an den Seitenflächen Lichtleiter eingesetzt, bei denen Licht auch seitwärts austreten kann, so dass der Operateur bei Benutzung einer derartigen bipolaren Elektrodenanordnung längs der Länge der Elektroden 2, 4 Lichtsignale erkennt, die die Position der Elektroden 2, 4 über ihre Länge hinweg anzeigen.

In Fig. 14 ist eine der Fig. 13 entsprechende Ausführungsform dargestellt, bei der zwei stabförmige, selbsttragende metallische Stabprofile 76, welche im Querschnitt die Form von Rohrwandungsabschnitten aufweisen, auf die äußere Ummantelung eines Lichtwellenleiters in Längsrichtung aufgebracht und dort befestigt sind und die Elektroden 2, 4 bilden. Der Lichtwellenleiter 16 besitzt in der dargestellten Form zwischen der Außenummantelung 61 und dem Kern 64 noch einen Mantel 62.

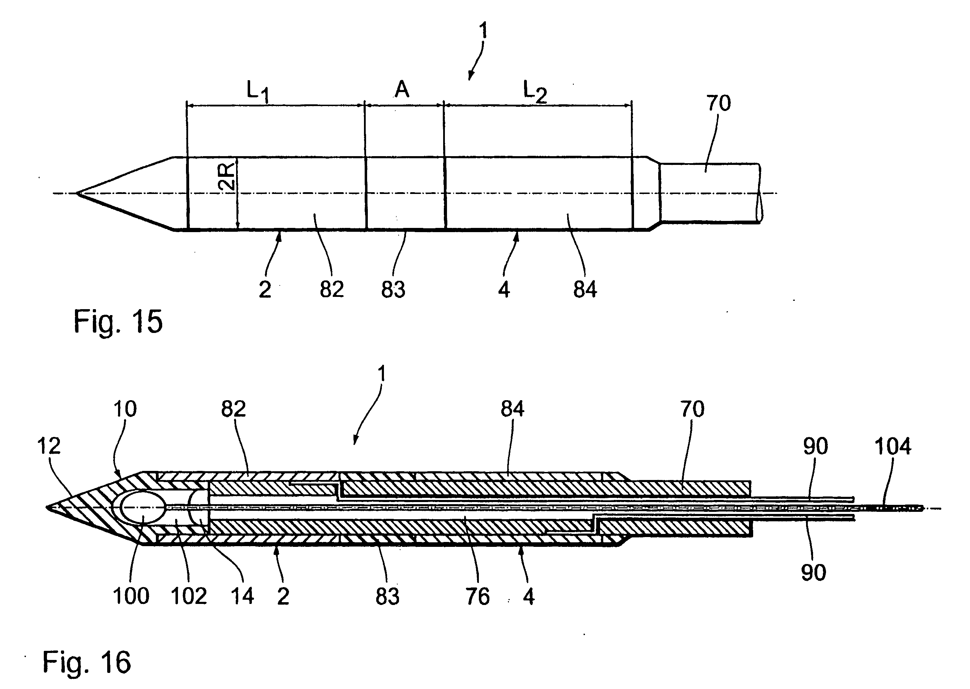

In den Fig. 15 und 16 ist eine Ausführungsform einer bipolaren Elektrodenanordnung 1 dargestellt, bei der ein Frontzylinder 10 am distalen Ende des Instruments mit einer distalen kegelförmigen Spitze 12 versehen ist, an den sich ein länglicher, elektrisch isolierender Träger 70 anschließt. Auf dem Träger 70 sind über ein Distanzstück 83 aus isolierendem Material zwei Rohrabschnitte 82, 84 aufgescho-ben und befestigt, welche zylindrische Elektroden 2, 4 bilden. Durch den Träger 70 verläuft axial hindurch ein zentraler Hohlkanal 76, der in eine Ausnehmung 14 in dem Frontzylinder 10 mündet und einen Temperatursensor 100 aufnimmt, der von einem Kunststoff- oder Kleberbett 102 umgeben und dort befestigt ist und über eine Leitung 104 ein Signal an das proximale Ende des Instruments abgibt, welches der Temperatur des Frontzylinders 10 entspricht. Vorgesehen sind ferner im Hohlkanal noch Anschlussleitungen 90, die - durch den Träger 70 hindurch - mit den Elektroden 2, 4 verbunden und an den proximalen HF-Generator anschließbar sind.

Die axiale Länge L1 der ersten Elektrode 2 und die axiale Länge L2 der zweiten Elektrode 4 sind - in allen dargestellten Ausführungsformen - größer als der Abstand A zwischen den beiden Elektroden 2, 4. Der Abstand A liegt bevorzugt in der Größenordnung des Außendurchmessers der Elektroden 2, 4. Das Isolierstück 83 besitzt denselben Außendurchmesser D =- 2R wie die Rohrabschnitte 82, 84, welche die Elektroden 2, 4 bilden. R ist der Radius der in der Regel kreiszylindrischen Elektrodenanordnung. In der Ausführungsform, die in Fig. 15 und 16 gezeigt ist, ist in einem besonders bevorzugten Fall L1 = L2.

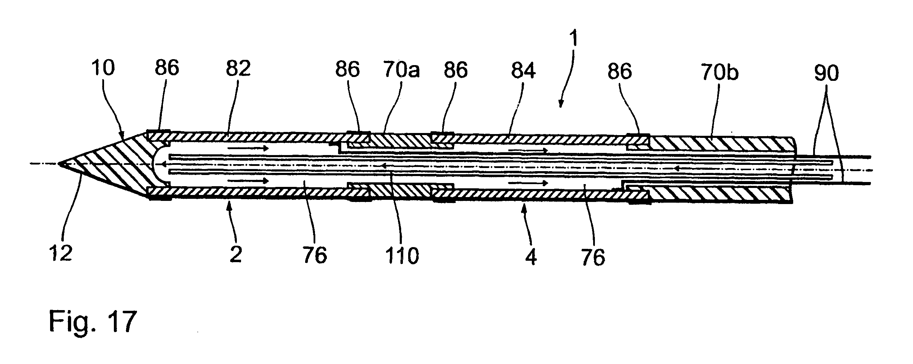

In Fig. 17 ist eine Ausführungsform einer bipolaren Elektrodenanordnung dargestellt, bei der ein Frontzylinder 10 mit einer kegelförmigen distalen Spitze 12 am Ende eines ersten Rohrabschnittes 82 befestigt ist, wobei dieser erste Rohrabschnitt 82 mit einem Endabschnitt an einem ersten Träger 70a befestigt ist. Ein zweiter Rohrabschnitt 84 sitzt mit einem Endabschnitt an dem ersten Träger 70a und mit seinem anderen Endabschnitt an einem zweiten Träger 70b. Die Träger 70a und 70b besitzen je einen Hohlkanal 76, dessen Achse mit der Achse der Rohrabschnitte 82, 84 fluchtet. Die Rohrabschnitte 82, 84 stellen die zylindrischen Elektroden 2, 4 dar.

Durch den Hohlkanal 76 der Träger 70a, 70b und der Rohrabschnitte 82, 84 ist ein Spülschlauch 110 hindurchgeführt, der Flüssigkeit an seinem distalen Ende abgibt, die dann in Kontakt mit der Innenwand der Rohrabschnitte 82, 84 durch den Hohlkanal 76 zum proximalen Ende zurückströmt und die beiden Rohrabschnitte 82, 84 kühlt. An den Stellen, wo die Rohrabschnitte über die isolierenden Träger 70a, 70b und über den isolierenden Frontzylinder 10 geschoben und an dieser Stelle festgeklebt sind, sind die Rohrabschnitte 82, 84 auf ihrer Außenfläche mit einer ringförmigen Isolationsschicht versehen, damit an diesen Stellen, die durch die Flüssigkeit nur geringer gekühlt werden, keine unerwünschte Überhöhung der Elektrodentemperatur erfolgt, welche zu einem unerwünschten Festbacken des Gewebes an den Elektroden oder zur Austrocknung des anliegenden Gewebes, und damit zu einem Impedanzanstieg in diesem Gewebe und zu einer Unterbrechung des elektrischen Stromes durch das Gewebe kommt.

Die Kühlung mit einer entsprechenden Kühlflüssigkeit hat zur Folge, dass das Gewebe an der Kontaktfläche zu den Elektroden eine vorgegebene Temperatur nicht überschreitet, und dass der Hot-Spot der Koagulation einige Millimeter entfernt von den Elektroden vorliegt.

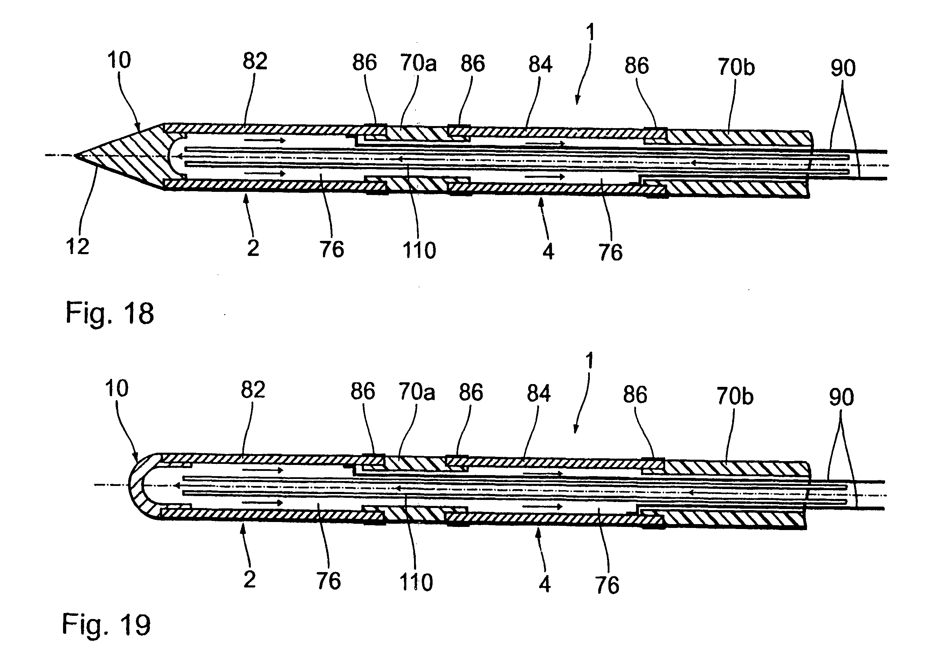

Die Fig. 18 und 19 entsprechen der Ausführungsform gemäß Fig. 17, wobei jedoch der Frontzylinder 10 in Fig. 18 und 19 aus Metall besteht und unmittelbar in den ersten Rohrabschnitt 82, die erste Elektrode, übergeht, und wobei der Frontzylinder 10 in Fig. 19 frontal abgerundet ist. In einer besonders bevorzugten Ausführungsform werden die Längen L1 und L2 der Elektroden 2, 4 so bemessen, daß die aus Frontzylinder und erster Elektrode gebildete Fläche gleich oder kleiner ist als die Oberfläche der zweiten Elektrode.

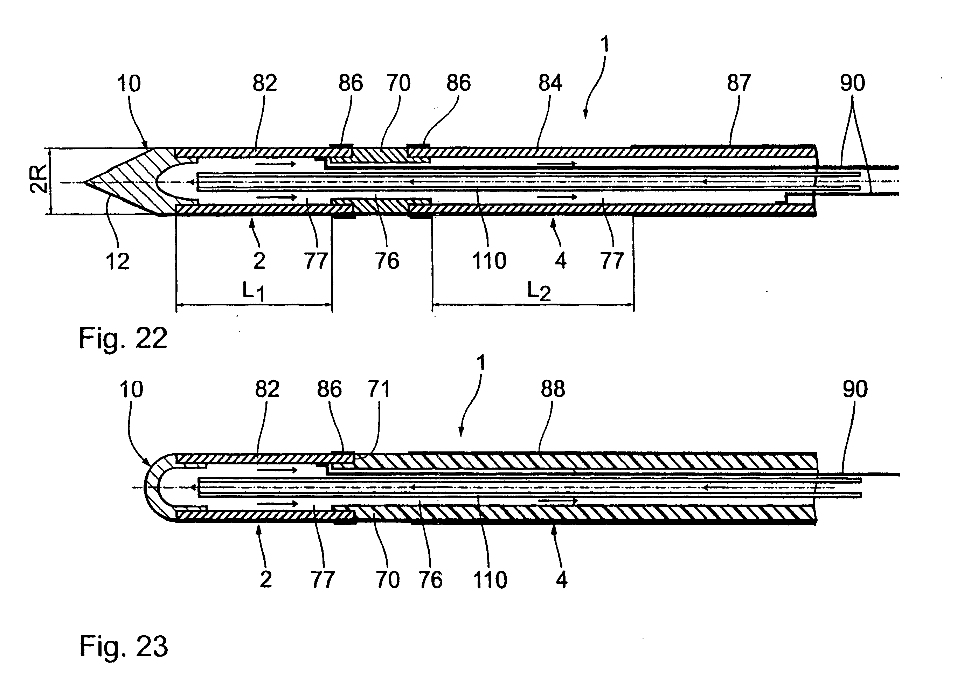

In Fig. 22 ist eine Ausführungsform der bipolaren Elektrodenanordnung dargestellt, die weitgehend derjenigen der Fig. 17 entspricht, wobei jedoch der zweite Rohrabschnitt 84, der die zweite Elektrode 4 darstellt, wesentlich länger als der erste Rohrabschnitt 82, der die erste Elektrode 2 darstellt, ist. Bei dieser Ausführungsform endet der zweite Rohrabschnitt 84 am proximalen Ende des Instruments, auf einen weiteren Träger am Ende des proximalen Instruments kann daher verzichtet werden. Die axiale Länge der zweiten Elektrode 4 wird dadurch begrenzt, dass auf dem proximalen Endabschnitt des zweiten Rohrabschnitts 84 eine Isolationsschicht 87 aufgebracht ist, die die Metalloberfläche des zweiten Rohrabschnittes 84 an dieser Stelle beispielsweise mittels einer Keramikbeschichtung abdeckt. Das Feld, welches die thermoelektrische Koagulation erzeugt, bildet sich dann zwischen den metallischen, zylindrischen Elektroden 2, 4 aus. Auch in dieser Ausführungsform sind die Endabschnitte der Rohrabschnitte 82, 84, welche innenseitig entweder von dem isolierenden Material des Frontzylinders 10 oder dem Isoliermaterial des Trägers 70 umgeben sind, auch auf der Außenfläche mit einer Isolierschicht 86 beschichtet, um an diesen Stellen - wegen der mangelnden Kühlung - eine punktuelle Überhitzung der Elektroden zu verhindern.

In Fig. 23 ist eine weitere Ausführungsform dargestellt, bei der ein frontal abgerundeter Frontzylinder 10 in einen ersten Rohrabschnitt 82 übergeht, der mit-seinem proximalen Ende an einem isolierenden Träger befestigt ist. In vorgegebenem Abstand von dem Rohrabschnitt 82 ist auf dem isolierenden Träger eine Metallschicht 88 vorgesehen. Das Rohr 82 bildet die zylindrische erste Elektrode 2, die Metallbeschichtung auf dem Träger 70 bildet die zylindrische zweite Elektrode 4. Träger 70 und Rohr 82 sind jeweils mit einem zentralen Hohlkanal 76, 77 ausgebildet, durch den - bis kurz vor das distale Ende des Frontzylinders 10 - ein Spülschlauch 110 hindurchläuft, der Flüssigkeit am distalen Ende abgibt, die in Kontakt mit der Innenfläche der ersten Elektrode 2 die erste Elektrode kühlt. Auf der Außenfläche des proximalen Endabschnitts des Rohrs 82 ist eine Isolierschicht 86 gelegt, um eine lokale Überhitzung der Elektrode 2 an dieser Stelle zu verhindern, da diese Stelle innenseitig nicht von Flüssigkeit kontaktiert wird. Der Frontzylinder 10 ist aus Metall gebildet, seine Abrundung am distalen Ende eignet sich vorteilhaft zur Behandlung von Randtumoren, bei der sich eine Schicht koagulierten Gewebes vor dem distalen Ende des Instruments bildet.

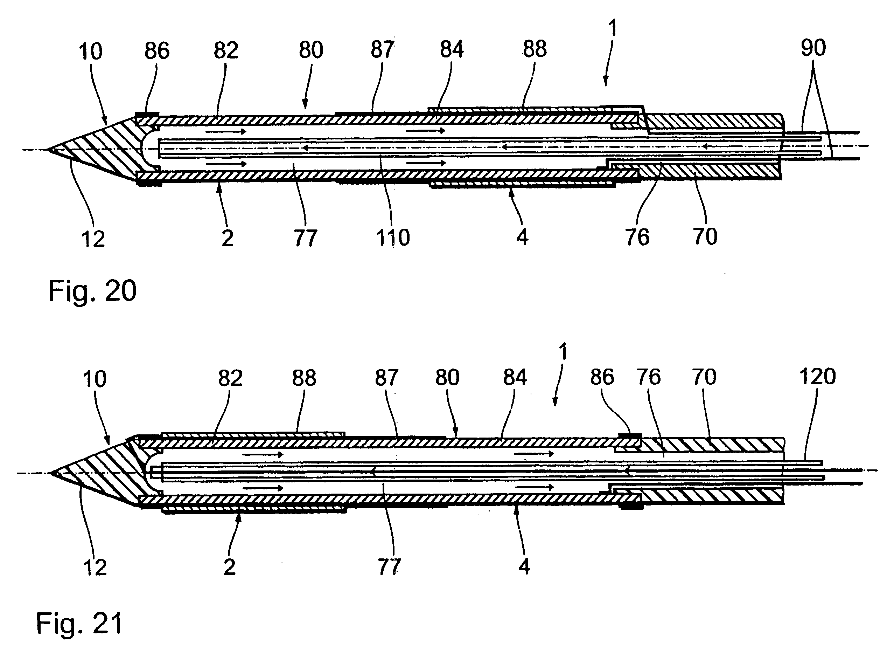

In den Fig. 20 und 21 sind weitere Ausführungsformen einer bipolaren Elektrodenanordnung angegeben. Der Grundaufbau sieht in beiden Fällen einen Frontzylinder 10 aus Metall oder isolierendem Material vor, an den sich ein Rohr 82 anschließt, das mit dem proximalen Endabschnitt an einem Träger 70 befestigt ist. Der Träger 70 und das Rohr 72 besitzen je einen zentralen Hohlkanal 76 bzw. 77, durch den ein Spülschlauch 110 geführt, der an seinem distalen Ende Flüssigkeit abgibt, die in Kontakt mit der Innenfläche des Rohrs 82 dann zum proximalen Ende des Instruments zurückläuft. In Fig. 18 besitzt der distale Rohrabschnitt 82 eine metallische Außenfläche und bildet die zylindrische erste Elektrode 2, der daran angrenzende proximale Rohrabschnitt 84 ist mit einer Isolierschicht 87 versehen, auf die am proximalen Ende eine Metallschicht 88 aufgebracht ist, welche die zylindrische zweite Elektrode 4 bildet. Bei der Ausführungsform gemäß Fig. 19 ist dagegen der distale Rohrabschnitt 82 mit einer Isolierschicht 87 versehen, auf die - am distalen Ende - eine Metallschicht 88 aufgebracht ist, welche die erste Elektrode bildet; bei dieser Ausführungsform bildet der proximale Rohrabschnitt die zweite Elektrode 4.

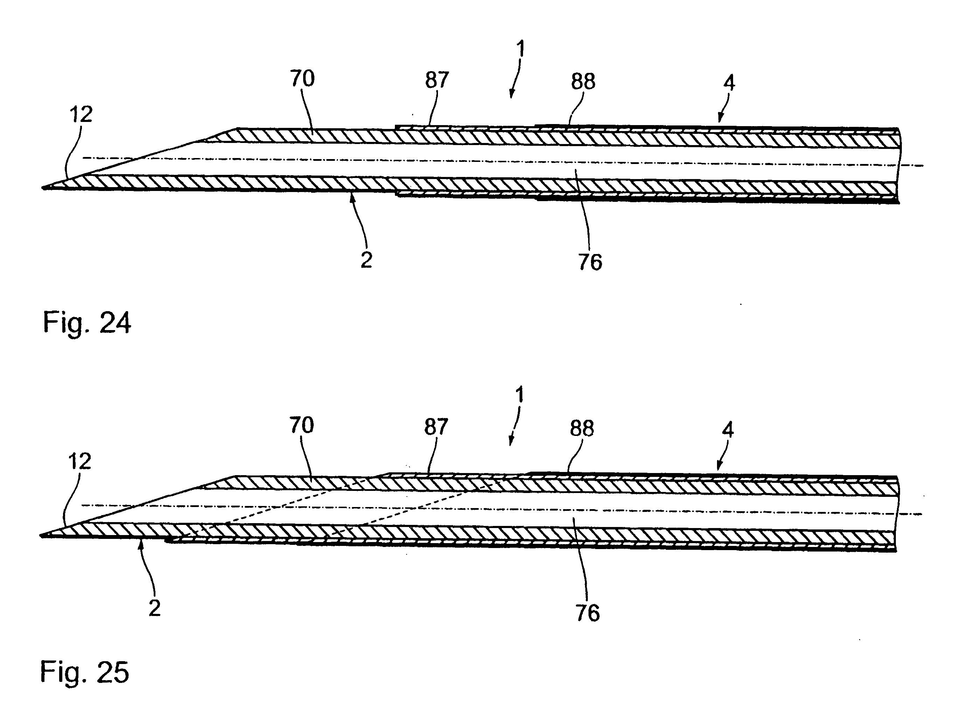

In Fig. 24 ist eine weitere Ausführungsform einer bipolaren Elektrodenanordnung dargestellt, bei der ein Träger 70 aus einem Metallrohr bis zu einer keilförmigen distalen Spitze 12 verläuft und die Form einer Kanüle besitzt. In vorgegebenem Abstand von der distalen Spitze 12 ist eine Isolierschicht 87 auf den Träger 70 aufgebracht, die in ihrem proximalen Bereich mit einer Metallbeschichtung 88 versehen ist. An die distale Spitze 12 schließt sich - mit metallischer Außenfläche - die zylindrische erste Elektrode 2 an, die Metallbeschichtung 88 am proximalen Bereich bildet die zylindrische zweite Elektrode 4. Durch das Rohr verläuft hindurch ein Hohlkanal 76, der am distalen Ende offen ist und zum Einspülen von Medikamenten dienen kann.

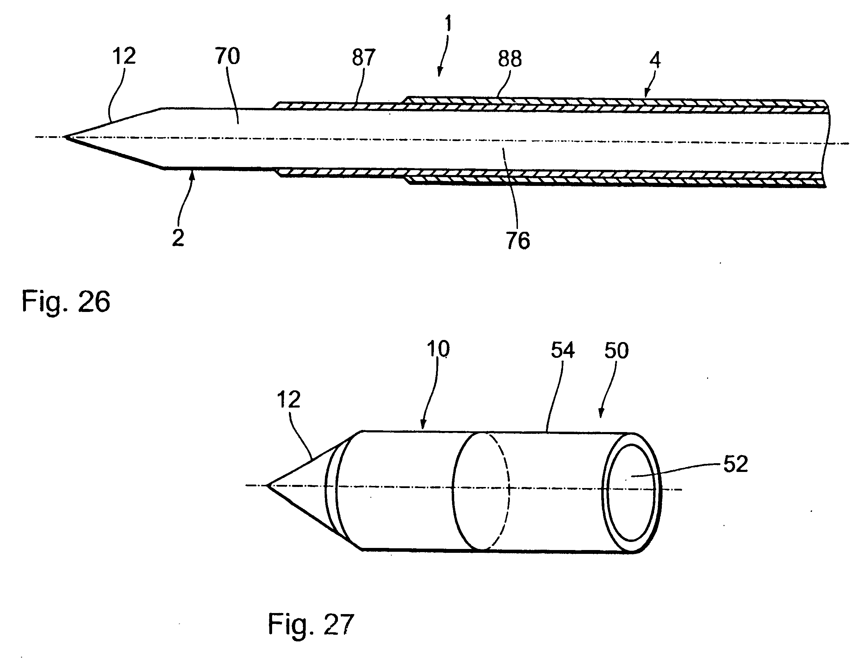

Die Fig. 25 und 26 stellen Varianten der Ausführungsform dar, die in Fig. 24 gezeigt ist. Die Ausführungsform gemäß Fig. 25 weicht von derjenigen der Fig. 24 insoweit ab, als die auf dem Metallträger 70 aufgebrachte Isolierschicht 87 und die auf der Isolierschicht 87 aufgebrachten Metallschicht 88 je eine distale Kante besitzen, die der keilförmigen distalen Spitze 12 des Trägers 70 entspricht.

In der Ausführungsform gemäß Fig. 26 wird als Träger 70 ein metallischer Stab verwendet (statt dem rohrförmigen Träger 70 der Fig. 24), der eine kegelförmige distale Spitze besitzt, und auf den die Isolierschicht 87 aufgebracht ist, auf der anschließend die Metallschicht 88 angeordnet ist.

Alle dargestellten Ausführungsformen der bipolaren Elektrodenanordnung 1 besitzen im wesentlichen einen Kreisquerschnitt mit Radius R und über ihre Länge hinweg einen möglichst homogenen Querschnitt. Unstetigkeiten im Außendurchmesser sind möglichst gering gehalten, um die Elektrodenanordnung leichtgleitend in das Gewebe einführen zu können.

Die axiale Länge der Elektroden ist in allen dargestellten Ausführungsformen größer als der Abstand der Elektroden, der im wesentlichen in der Größenordnung des Außendurchmessers liegt. Bei dieser Dimensionierung ist eine vorteilhafte lokale Konzentration des Koagulierungsvorganges und eine ausreichende Stärke des elektrischen Feldes gegeben. Der Träger 70 kann entweder flexibel oder starr ausgebildet sein.

In allen Ausführungsbeispielen, bei denen ein Spülschlauch Spülflüssigkeit am distalen Ende eines Hohlraumes abgibt, besteht die Möglichkeit, die Elektroden und den Träger vor dem Einführen der Elektrodenanordnung in den Körper zu temperieren, d.h. über 30°C, bevorzugt über 50°C zu erwärmen. Dadurch lässt sich die Elektrodenanordnung leichter in das Gewebe einführen. Sobald dann die Elektrodenanordnung den Behandlungsort erreicht hat und die eigentliche elektrothermische Behandlung eingeleitet werden soll, wird zur Erzielung eines optimalen Koagulatiönsverlaufes das Instrument gekühlt, um ein Austrocknen des Gewebes an den Elektroden zu verhindern, und um höhere Leistungen, und stärkere elektrische Felder applizieren zu können, ohne dass das Gewebe an den Elektroden festbackt.

Fig. 27 zeigt einen Frontzylinder 10, der beispielsweise aus Aluminium oder Titan oder einer Titanlegierung besteht und durch anodisches Oxidieren, also einen proximalen Endbereich mit einer Keramikschicht versehen wurde, die das Isolierelement 50 bildet, dessen Trennwand 52 an dem proximalen Ende des Frontzylinders ausgebildet ist, und dessen Hüllwand 54 sich außen um einen axialen Längenabschnitt des Frontzylinders hin erstreckt.

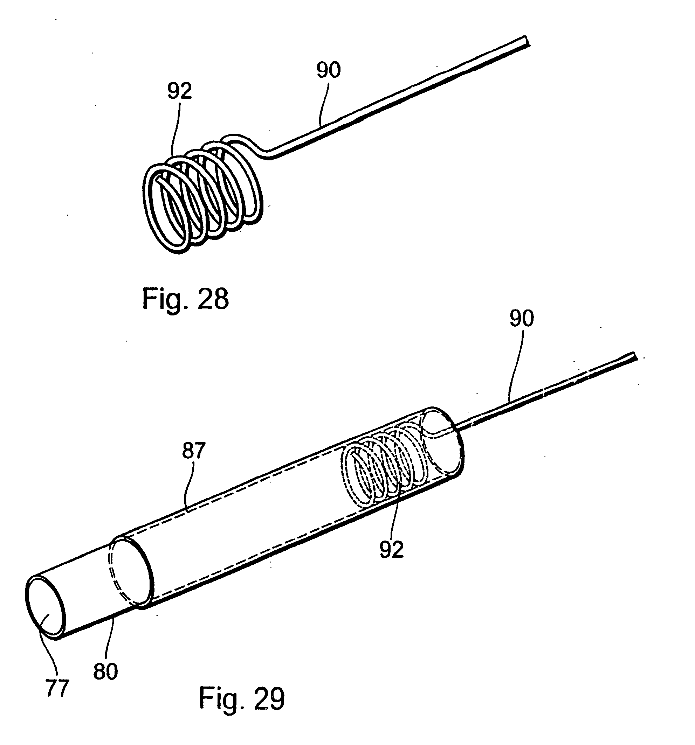

Die Fig. 28 und 29 zeigen einen als Spiralfeder 92 ausgebildeten Endabschnitt einer Anschlussleitung 90, die gemäß Fig. 25 in den Hohlkanal 77 eines Metallrohres 80 eingesetzt ist und unter radialem Andruck gegen die Innenfläche des Rohres 80 anliegt und dieses kontaktiert. Auf dem Metallrohr 80 ist eine Isolierschicht 87 dargestellt.

## Patentansprüche

1. Elektrodenanordnung für ein chirurgisches Instrument zur elektrothermischen Koagulation im Gewebe, enthaltend
einen elektrisch leitenden Frontzylinder (10) am distalen Ende des Instruments, mit einer distalen Spitze (12), und mit einer zylindrischen ersten Elektrode (2),
einen proximal an den Frontzylinder anschließenden rohrförmigen Außenleiter (20) mit einer zylindrischen zweiten Elektrode (4),
ein Isolationselement (50) zwischen dem Frontzylinder (10) und dem Außenleiter (20),
wobei die Elektroden (2, 4) an eine Wechselspannungsquelle anschließbar sind,
einen stabförmigen Innenleiter (40) in dem Außenleiter (20) und einen Isolationsschlauch (30) zwischen dem Innenleiter (40) und dem Außenleiter (20), **dadurch gekennzeichnet, dass** das Isolationselement (50) als Ringkörper (58) mit einer vorgegebenen axialen Länge ausgebildet ist, durch den der Innenleiter (40) hindurchläuft, und dass der Frontzylinder (10) und der Außenleiter (20) an den Stirnseiten des Ringkörpers anliegen.

2. Elektrodenanordnung für ein chirurgisches Instrument nach Anspruch 1, **dadurch gekennzeichnet, dass** der Ring Körper an seinem äußeren Rand in eine zylindrische Hüllwand (54) übergeht, welche den Frontzylinder (10) und/oder den Außenleiter (20) eng anliegend über einen vorgegebenen axialen Längsabschnitt umgibt.

3. Elektrodenanordnung für ein chirurgisches Instrument nach einem der vorstehenden Ansprüche, **dadurch gekennzeichnet, dass** der Außenleiter (20) und der Frontzylinder (10) im wesentlichen denselben Außendurchmesser aufweisen.

4. Elektrodenanordnung für ein chirurgisches Instrument nach einem der vorstehenden Ansprüche, **dadurch gekennzeichnet, dass** der Frontzylinder (10) mittels einer Schraubverbindung (14) mit dem Innenleiter (40) lösbar verbindbar ist.

5. Elektrodenanordnung für ein chirurgisches Instrument nach Anspruch 4, **dadurch gekennzeichnet, dass** zur Bildung der Schraubverbindung (14) der Innenleiter (40) an seinem distalen Ende ein Außengewinde trägt, welches mit einem entsprechenden Innengewinde im Frontzylinder (10) verschraubbar ist.

6. Elektrodenanordnung für ein chirurgisches Instrument nach einem der vorstehenden Ansprüche, **dadurch gekennzeichnet, dass** der Isolationsschlauch (30) distal mit dem Außenleiter (20) endet und gegen den Ring Körper anliegt.

7. Elektrodenanordnung für ein chirurgisches Instrument nach einem der vorstehenden Ansprüche, **dadurch gekennzeichnet, dass** der Innenleiter (40), der Außenleiter (20), das Isolationselement (50) und der Isolationsschlauch (30) aus elastischem Material bestehen und biegsam sind.

8. Elektrodenanordnung für ein chirurgisches Instrument nach einem der vorstehenden Ansprüche, **dadurch gekennzeichnet, dass** der Innenleiter (40) und der Außenleiter (20) starr ausgebildet sind.

9. Elektrodenanordnung für ein chirurgisches Instrument nach Anspruch 3, **dadurch gekennzeichnet, dass** der Frontzylinder (10) aus Titan oder Aluminium besteht.

10. Elektrodenanordnung für ein chirurgisches Instrument nach einem der vorstehenden Ansprüche, **dadurch gekennzeichnet, dass** der Außenleiter (20) und der Frontzylinder (10) über ihre axiale Länge gerade verlaufen und koaxial fluchtend zueinander angeordnet sind.

11. Elektrodenanordnung für ein chirurgisches Instrument nach einem der vorstehenden Ansprüche, **dadurch gekennzeichnet, dass** das Instrument in Längsrichtung abgewinkelt verläuft.

12. Elektrodenanordnung für ein chirurgisches Instrument nach Anspruch 2, **dadurch gekennzeichnet, dass** der Außenleiter (20) über den vollen Längenabschnitt, der nicht von der zylindrischen Hüllwand bedeckt ist, die zweite zylindrische Elektrode (4) bildet.

13. Elektrodenanordnung für ein chirurgisches Instrument nach Anspruch 2, **dadurch gekennzeichnet, dass** der Frontzylinder (10) über seinen vollen Längenabschnitt, der nicht von der zylindrischen Hüllwand bedeckt ist, die erste Elektrode (2) bildet.

14. Elektrodenanordnung für ein chirurgisches Instrument nach einem der vorstehenden Ansprüche, **dadurch gekennzeichnet, dass** der Innenleiter (40) und der Frontzylinder (10) einen zentralen Hohlkanal aufweisen, der aus der distalen Spitze (12) des Frontzylinders (10) austritt und einen Lichtwellenleiter (60) enthält, der mit Laser-Signalen beaufschlagbar ist.

15. Elektrodenanordnung für ein chirurgisches Instrument nach einem der vorstehenden Ansprüche, **dadurch gekennzeichnet, dass** der Frontzylinder (10) oder ein proximal angrenzender Längenabschnitt des Instruments aus lichtdurchlässigem oder teilweise lichtdurchlässigem Material besteht und in einer zentralen Ausnehmung eine Lichtquelle enthält, die Streulicht nach außen abgibt.

16. Elektrodenanordnung für ein chirurgisches Instrument nach einem der vostehenden Ansprüche, **dadurch gekennzeichnet, dass** der isolierende Ringkörper (58) aus lichtdurchlässigem oder teilweise lichtdurchlässigem Material besteht.

17. Elektrodenanordnung für ein chirurgisches Instrument nach Anspruch 16, **dadurch gekennzeichnet, dass** in dem isolierenden Ringkörper (58) eine Lichtquelle angeordnet ist, deren Licht durch den isolierenden Ringkörper (58) hindurch als Streulicht austritt.

18. Elektrodenanordnung für ein chirurgisches Instrument nach einem der vorstehenden Ansprüche, **dadurch gekennzeichnet, dass** die Spitze (12) des Frontzylinders (10) kegelförmig ausläuft.

19. Elektrodenanordnung für ein chirurgisches Instrument nach einem der Ansprüche 1 bis 17, **dadurch gekennzeichnet, dass** die Spitze (12) des Frontzylinders (10) keilförmig ausläuft.

20. Elektrodenanordnung für ein chirurgisches Instrument nach einem der vorstehenden Ansprüche, **dadurch gekennzeichnet, dass** die axiale Länge der Elektroden (2, 4) größer ist als der Durchmesser der Elektroden (2, 4).

21. Elektrodenanordnung für ein chirurgisches Instrument nach einem der vorstehenden Ansprüche, **dadurch gekennzeichnet, dass** die axiale Länge der Elektroden (2, 4) größer ist als die axiale Länge des vom Isolationselement (50) belegten Längenabschnitts.

22. Elektrodenanordnung für ein chirurgisches Instrument nach einem der vorstehenden Ansprüche, **dadurch gekennzeichnet, dass** die axiale Länge der Elektroden (2, 4) größer ist als der Außendurchmesser des Frontzylinders (10) bzw. des Außenleiters (20).

23. Elektrodenanordnung für ein chirurgisches Instrument nach einem der vorstehenden Ansprüche, **dadurch gekennzeichnet, dass** der axiale Abstand der Elektroden (2, 4) voneinander etwa gleich oder kleiner ist als der Außendurchmesser des Frontzylinders (10).

24. Elektrodenanordnung für ein chirurgisches Instrument nach einem der vorstehenden Ansprüche, **dadurch gekennzeichnet, dass** das bzw. die selbsttragenden Metallrohre oder Rohrabschnitte (70, 80, 82, 84) und/oder der metallische Zylinderkörper (10), die zur Ausbildung der Elektroden (2, 4) vorgesehen sind, aus Titan oder Aluminium bestehen.

## Claims

1. Electrode arrangement for a surgical instrument for thermoelectric coagulation in tissue, including an electrically conductive front cylinder (10) at the distal end of the instrument, with a distal tip (12) and with a cylindrical first electrode (2), a tubular outer conductor (20) proximally adjoining the front cylinder and with a cylindrical second electrode (4), an insulator element (50) between the front cylinder (10) and the outer conductor (20), wherein the electrodes (2, 4) are connectable to an AC voltage source, a bar-shaped inner conductor (40) in the outer conductor (20) and an insulating tube (20) between the inner conductor (40) and the outer conductor (20), **characterised in that** the insulator element (50) is configured as an annular body (58) having a predetermined axial length, through which the inner conductor (40) passes, and **in that** the front cylinder (10) and the outer conductor (20) abut the end faces of the annular body.

2. Electrode arrangement for a surgical instrument according to claim 1, **characterised in that** the outer edge of the annular body merges into a cylindrical casing wall (54) which surrounds the front cylinder (10) and/or the outer conductor (20) in a closely fitting relationship therewith over a predetermined axial longitudinal portion.

3. Electrode arrangement for a surgical instrument according to any one of the preceding claims, **characterised in that** the outer conductor (20) and the front cylinder (10) are of substantially the same external diameter.

4. Electrode arrangement for a surgical instrument according to any one of the preceding claims, **characterised in that** the front cylinder (10) is detachably connectable to the inner conductor (40) by means of a screw connection (14).

5. Electrode arrangement for a surgical instrument according to any one of the preceding claims, **characterised in that**, in order to form the screw connection (14), the inner conductor (40) carries at its distal end a male screw thread which can be screwed to a corresponding female screw thread in the front cylinder (10).

6. Electrode arrangement for a surgical instrument according to any one of the preceding claims, **characterised in that** the insulating tube (30) ends distally with the outer conductor (20) and abuts the annular body.

7. Electrode arrangement for a surgical instrument according to any one of the preceding claims, **characterised in that** the inner conductor (40), the outer conductor (20), the insulator element (50 and the insulating tube (30) are made of a resilient material and are pliable.

8. Electrode arrangement for a surgical instrument according to any one of the preceding claims, **characterised in that** the inner conductor (40) and the outer conductor (20) are of a rigid configuration.

9. Electrode arrangement for a surgical instrument according to claim 3, **characterised in that** the front cylinder (10) is made of titanium or aluminium.

10. Electrode arrangement for a surgical instrument according to any one of the preceding claims, **characterised in that** the outer conductor (20) and the front cylinder (10) are straight over their axial length and are configured in a mutually coaxially aligned arrangement.

11. Electrode arrangement for a surgical instrument according to any one of the preceding claims, **characterised in that** the instrument is angled in the longitudinal direction.

12. Electrode arrangement for a surgical instrument according to claim 2, **characterised in that** the outer conductor (20), over the entire longitudinal portion which is not covered by the cylindrical casing wall, forms the second cylindrical electrode (4).

13. Electrode arrangement for a surgical instrument according to claim 2, **characterised in that** the front cylinder (10), over its entire longitudinal portion which is not covered by the cylindrical casing wall, forms the first electrode (2).

14. Electrode arrangement for a surgical instrument according to any one of the preceding claims, **characterised in that** the inner conductor (40) and the front cylinder (10) comprise a central hollow duct which issues from the distal tip (12) of the front cylinder (10) and contains an optical waveguide (60) which can be acted on with laser signals.

15. Electrode arrangement for a surgical instrument according to any one of the preceding claims, **characterised in that** the front cylinder (10) or a proximally adjoining longitudinal portion of the instrument is made of translucent or partially translucent material and contains in a central recess a light source which discharges stray light outwardly.

16. Electrode arrangement for a surgical instrument according to any one of the preceding claims, **characterised in that** the insulating annular body (58) is made of translucent or partially translucent material.

17. Electrode arrangement for a surgical instrument according to claim 6, **characterised in that** disposed in the insulating annular body (58) is a light source, the light of which issues outwardly through the insulating annular body (58) as stray light.

18. Electrode arrangement for a surgical instrument according to any one of the preceding claims, **characterised in that** the tip (12) of the front cylinder (10) terminates in a conical configuration.

19. Electrode arrangement for a surgical instrument according to any one of claims 1 to 17, **characterised in that** the tip (12) of the front cylinder (10) terminates in a wedge-shaped configuration.

20. Electrode arrangement for a surgical instrument according to any one of the preceding claims, **characterised in that** the axial length of the electrodes (2, 4) is greater than the diameter of the electrodes (2, 4).

21. Electrode arrangement for a surgical instrument according to any one of the preceding claims, **characterised in that** the axial length of the electrodes (2, 4) is greater than the axial length of the longitudinal portion covered by the insulator element (50).

22. Electrode arrangement for a surgical instrument according to any one of the preceding claims, **characterised in that** the axial length of the electrodes (2, 4) is greater than the external diameter of the front cylinder (10) or of the outer conductor (20).

23. Electrode arrangement for a surgical instrument according to any one of the preceding claims, **characterised in that** the axial spacing of the electrodes (2, 4) from one another is approximately equal to or smaller than the external diameter of the front cylinder (10).

24. Electrode arrangement for a surgical instrument according to any one of the preceding claims, **characterised in that** the self-supporting metal tube or tube portions (70, 80, 82, 84) and/or the metal cylinder body (10), which are provided for forming the electrodes (2, 4), are made of titanium or aluminium.

## Revendications

1. Dispositif d'électrode pour un instrument chirurgical en vue de la coagulation électrothermique d'un tissu, comprenant
un cylindre frontal électriquement conducteur (10) à une extrémité distale de l'instrument, équipé d'une pointe distale (12), et d'une première électrode cylindrique (2),
un conducteur extérieur (20) tubulaire proximal connecté au cylindre frontal muni d'une seconde électrode cylindrique (4),
un élément isolant (50) entre le cylindre frontal (10) et le conducteur extérieur (20),
dans lequel les électrodes (2, 4) peuvent être reliées à une source de courant alternatif,
un conducteur interne en forme de barre (40) dans le conducteur extérieur (20) et un tuyau flexible isolant (30) entre le conducteur interne (40) et le conducteur extérieur (20), **caractérisé en ce que** l'élément isolant (50) est réalisé sous la forme d'un corps annulaire (58) avec une longueur axiale prédéterminée, à travers lequel le conducteur interne (40) se déplace, et **en ce que** le cylindre frontal (10) et le conducteur extérieur (20) viennent en contact sur les côtés antérieurs du corps annulaire.

2. Dispositif d'électrode pour un instrument chirurgical selon la revendication 1, **caractérisé en ce que** le corps annulaire se transforme sur son bord extérieur en une paroi enveloppante cylindrique (54), qui entoure de façon étroitement adjacente le cylindre frontal (10) et/ou le conducteur extérieur (20) sur une section longitudinale axiale prédéterminée.

3. Dispositif d'électrode pour un instrument chirurgical selon l'une des revendications précédentes, **caractérisé en ce que** le conducteur extérieur (20) et le cylindre frontal (10) présentent sensiblement le même diamètre externe.

4. Dispositif d'électrode pour un instrument chirurgical selon l'une des revendications précédentes, **caractérisé en ce que** le cylindre frontal (10) peut être relié de façon amovible au conducteur interne (40) par vissage (14).

5. Dispositif d'électrode pour un instrument chirurgical selon la revendication 4, **caractérisé en ce que**, en vue de la formation du vissage (14), le conducteur interne (40) présente un filetage à son extrémité distale, qui peut être vissé dans un taraudage correspondant dans le cylindre frontal (10).

6. Dispositif d'électrode pour un instrument chirurgical selon l'une des revendications précédentes, **caractérisé en ce que** le tuyau flexion isolant (30) se termine côté distal par le conducteur extérieur (20) et vient en contact contre le corps annulaire.

7. Dispositif d'électrode pour un instrument chirurgical selon l'une des revendications précédentes, **caractérisé en ce que** le conducteur interne (40), le conducteur extérieur (20), l'élément isolant (50) et le tuyau flexible isolant (30) sont composés d'un matériau élastique et peuvent être courbés.

8. Dispositif d'électrode pour un instrument chirurgical selon l'une des revendications précédentes, **caractérisé en ce que** le conducteur interne (40) et le conducteur extérieur (20) sont réalisés de façon rigide.

9. Dispositif d'électrode pour un instrument chirurgical selon la revendication 3, **caractérisé en ce que** le cylindre frontal (10) se compose de titane ou d'aluminium.

10. Dispositif d'électrode pour un instrument chirurgical selon l'une des revendications précédentes, **caractérisé en ce que** le conducteur extérieur (20) et le cylindre frontal (10) se déplacent en ligne droite le long de leur longueur axiale et sont disposés alignés coaxialement l'un par rapport à l'autre.

11. Dispositif d'électrode pour un instrument chirurgical selon l'une des revendications précédentes, **caractérisé en ce que** l'instrument se déplace de façon inclinée dans la direction longitudinale.

12. Dispositif d'électrode pour un instrument chirurgical selon la revendication 2, **caractérisé en ce que** le conducteur extérieur (20) forme la seconde électrode cylindrique (4) sur toute la section longitudinale, qui n'est pas recouverte par la paroi enveloppante cylindrique.

13. Dispositif d'électrode pour un instrument chirurgical selon la revendication 2, **caractérisé en ce que** le cylindre frontal (10) forme la première électrode (2) sur toute sa section longitudinale, qui n'est pas recouverte par la paroi enveloppante cylindrique.

14. Dispositif d'électrode pour un instrument chirurgical selon l'une des revendications précédentes, **caractérisé en ce que** le conducteur interne (40) et le cylindre frontal (10) présentent un canal creux central, qui émerge de la pointe distale (12) du cylindre frontal (10) et comprend un guide d'ondes optique (60), qui est alimenté par des signaux laser.

15. Dispositif d'électrode pour un instrument chirurgical selon l'une des revendications précédentes, **caractérisé en ce que** le cylindre frontal (10) ou une section longitudinale adjacente proximale de l'instrument se compose d'un matériau transparent ou partiellement transparent et comporte une source lumineuse dans une cavité centrale, qui délivre une lumière diffuse vers l'extérieur.

16. Dispositif d'électrode pour un instrument chirurgical selon l'une des revendications précédentes, **caractérisé en ce que** le corps annulaire isolant (58) se compose d'un matériau transparent ou partiellement transparent.

17. Dispositif d'électrode pour un instrument chirurgical selon la revendication 16, **caractérisé en ce que**, dans le corps annulaire isolant (58), est disposée une source lumineuse, dont la lumière émerge du corps annulaire isolant (58) sous forme de lumière diffuse.

18. Dispositif d'électrode pour un instrument chirurgical selon l'une des revendications précédentes, **caractérisé en ce que** la pointe (12) du cylindre frontal (10) sort sous forme de cône.

19. Dispositif d'électrode pour un instrument chirurgical selon l'une des revendications 1 à 17, **caractérisé en ce que** la pointe (12) du cylindre frontal (10) sort sous forme de coin.

20. Dispositif d'électrode pour un instrument chirurgical selon l'une des revendications précédentes, **caractérisé en ce que** la longueur axiale des électrodes (2, 4) est supérieure au diamètre des électrodes (2,4).

21. Dispositif d'électrode pour un instrument chirurgical selon l'une des revendications précédentes, **caractérisé en ce que** la longueur axiale des électrodes (2, 4) est supérieure à la longueur axiale de la section longitudinale occupée par l'élément isolant (50).

22. Dispositif d'électrode pour un instrument chirurgical selon l'une des revendications précédentes, **caractérisé en ce que** la longueur axiale des électrodes (2, 4) est supérieure au diamètre externe du cylindre frontal (10) ou du conducteur extérieur (20).

23. Dispositif d'électrode pour un instrument chirurgical selon l'une des revendications précédentes, **caractérisé en ce que** la distance axiale des électrodes (2, 4) l'une de l'autre est sensiblement égale ou inférieure au diamètre externe du cylindre frontal (10).

24. Dispositif d'électrode pour un instrument chirurgical selon l'une des revendications précédentes, **caractérisé en ce que** le ou les tubes métalliques ou tronçons de tube autoporteurs (70, 80, 82, 84) et/ou le corps cylindre métallique (10), qui sont prévus en vue de la réalisation des électrodes (2, 4), se composent de titane ou d'aluminium.
